Europäisches Patentamt

European Patent Office

Office européen des brevets

(11) Publication number: **0 267 779**
A2

(12) # EUROPEAN PATENT APPLICATION

(21) Application number: **87309935.2**

(22) Date of filing: **10.11.87**

(51) Int. Cl.⁴: **C 12 P 21/02**
C 07 K 13/00, C 12 N 15/00,
A 61 K 37/02

(30) Priority: **10.11.86 US 928900   05.01.87 US 551**

(43) Date of publication of application:
**18.05.88   Bulletin   88/20**

(84) Designated Contracting States:
**AT BE CH DE ES FR GB GR IT LI LU NL SE**

(71) Applicant: **Schering Biotech Corporation**
**901 California Avenue**
**Palo Alto California 94304-1104  (US)**

(72) Inventor: **Coffman, Robert**
**239 Echo Lane**
**Portola Valley California 94025  (US)**

**Yokota, Takashi**
**890 Colorado Avenue**
**Palo Alto California 94303  (US)**

**Crute, James J.**
**422 College Avenue**
**Palo Alto California 94306  (US)**

**Lee, Frank**
**212 Rinconada Avenue**
**Palo Alto California 94301  (US)**

**Arai, Ken-ichi**
**648 Georgia Avenue**
**Palo Alto California 94306  (US)**

(74) Representative: **Ritter, Stephen David et al**
**Mathys & Squire 10 Fleet Street**
**London EC4Y 1AY  (GB)**

(54) Human pleiotropic immune factor and muteins thereof.

(57) A novel immune system mediator, termed pleiotropic immune factor (PIF) is provided which enhances the secretion of immunoglobulin, particularly IgA, and which promotes the growth and differentiation of eosinophils. The human PIF amino acid sequence is disclosed, and a synthetic gene is provided for cassette mutagenesis in a pcD plasmid. Mutant and native human PIFs are expressed in COS 7 monkey cells.

EP 0 267 779 A2

**Description**

## HUMAN PLEIOTROPIC IMMUNE FACTOR AND MUTEINS THEREOF

The present invention relates generally to protein and mutein factors of the mammalian immune system and to nucleic acids coding therefor. More particularly, the invention relates to a novel class of mammalian lymphokines (along with their encoding nucleic acids) which exhibit pleiotropic stimulatory effects on the immune system, including B cell differentiation factor activity and promotion of eosinophil colony formation and growth.

A large number of soluble glycoprotein factors mediate cellular growth- and differentiation-responses of the mammalian immune system. These factors are referred to as lymphokines, cytokines, monokines, hemopoietins, and the like; e.g. Metcalf, The Hemopoietic Colony Stimulating Factors (Elsevier, New York, 1984), and Sorg et al., Eds., Cellular and Molecular Biology of Lymphokines (Academic Press, New York, 1985).

The detection, isolation and purification of these factors is extremely difficult, for it is frequently complicated by the nature of the supernatants they are typically located in, the divergencies and cross-overs of activities of the various components in the mixtures, the sensitivity (or lack thereof) of the assays utilized to ascertain the factors' properties, the frequent similarity in the range of molecular weights and other characteristics of the factors, and the very low concentration of the factors in their natural settings.

As more lymphokines become available, primarily through molecular cloning, interest has heightened in finding clinical applications for them. Because of physiological similarities to hormones (e.g., soluble factors, growth mediators, action via cell receptors), potential uses of lymphokines have been analogized to the current uses of hormones; e.g. Dexter, Nature, Vol. 321, pg. 198 (1986). One hope is that the levels of lymphokines in a patient can be manipulated directly or indirectly to bring about a beneficial immune response, e.g. suppression of inflammation, of allergy, or of tissue rejection, or stimulation or potentiation in the case of infection or malignant growth. Other potential clinical uses of lymphokines include maintaining and expanding in vitro populations of certain immune system cells of one person for eventual reintroduction into the same or another person for a beneficial effect. For example, investigations are currently under way to determine whether populations of lymphokine-activated killer T cells of a patient can be expanded outside his or her body and then reinjected to bring about an enhanced antitumor response. Another potential clinical use of lymphokines, particularly of colony stimulating factors such as granulocyte-macrophage colony stimulating factor (GM-CSF), and factors which enhance their activities, is stimulating blood cell generation, for example in pre- or post-chemotherapy or radiation therapy against tumors, in treatment of myeloid hypoplasias, or in treatment of neutrophil deficiency syndromes: Dexter, Nature, Vol. 321, pg. 198 (1986). Another area where such factors would be useful is in bone marrow transplant therapy, which is being used increasingly to treat aplastic anemia and certain leukemias.

There are two properties of lymphokines that have important consequences for such clinical applications: Individual lymphokines are frequently pleiotropic. And the biological effects of one lymphokine can usually be modulated by at least one other lymphokine, either by inhibition or by potentiation. For example, tumor necrosis factor, which synergizes with gamma-interferon, stimulates interleukin-1 (IL-1) production and can activate the phagocytic activity of neutrophils. IL-1, a protein produced by activated macrophages, mediates a wide range of biological activities, including stimulation of thymocyte proliferation by induction of interleukin-2 (IL-2) release, stimulation of B-lymphocyte maturation and proliferation, and induction of acute-phase protein synthesis by hepatocytes, and also shows fibroblast growth factor activity. Interleukin-2, formerly referred to as T cell growth factor, is a lymphokine which is produced by lectin- or antigen-activated T cells. The reported biological activities of IL-2 include stimulation of the long-term in vitro growth of activated T cell clones, enhancement of thymocyte mitogenesis, induction of cytotoxic T cell reactivity and plaque-forming cell responses in cultures of nude mouse spleen cells, and B cell growth factor activity; and IL-2, in addition, like interferons (IFNs), has been shown to augment natural killer cell activity, suggesting a potential use in the treatment of neoplastic diseases: Henney et al., Nature, Vol. 291, pgs. 335-338 (1981), and Rosenberg and Lotze, "Cancer Immunotherapy Using Interleukin-2 and Interleukin-2-Activated Lymphocytes," Ann. Rev. Immunol., Vol. 4, pgs. 681-709 (1986).

In recent years there has been increased interest in factors which regulate B cell growth and differentiation: e.g., Bialy, Biotechnology, Vol. 4, pgs. 614-621 (1986), and the following reviews: Greenblatt and Howard, in Biological Response Modifiers and Cancer (Marcel Dekker, New York, 1987), Howard and Paul, Ann. Rev. Immunol., Vol. 1, pgs. 307-333 (1983), Howard et al., Immunol. Rev., 1984, No. 78, pgs. 185-210, Kishimoto et al., Immunol Rev., 1984, No. 78 pgs. 97-118, and Kishimoto, Ann. Rev. Immunol., Vol. 3, pgs. 133-157 (1985). One practical aim of this interest is the development of an alternative therapy to combat infections by selectively stimulating antibody production by use of one or more growth and/or differentiation factors, either alone or concomitantly with antibiotics.

Some confusion has existed over the nomenclature used for labeling the various B cell factors because of differences in source materials, difficulties in purification, and differences in the assays used to define their biological activities.

B cell growth factor (BCGF) activity is characterized by a capacity to cause DNA synthesis in B cells co-stimulated by exposure to anti-IgM or like antigens. It is believed that interleukin-1 is also required for BCGF activity to be manifested, at least when the assay is conducted with low densities of B cells. Alternative assays for human BCGF have been described, e.g. Maizel et al., Proc. Natl. Acad. Sci., Vol. 80, pgs. 5047-5051 (1983) (support of long-term growth of human B cells in culture). The BCGF activity associated with the former assay has also been variously labelled B cell stimulatory factor-1 (BSF-1) activity and BCGF I, to distinguish it from similar and/or related activities. Currently it is believed that BSF-1 (BCGF I) is identical to the factor designated interleukin-4 (IL-4), recently cloned and characterized by Lee et al., Proc. Natl. Acad. Sci., Vol. 83, pgs. 2061-2065 (1986), and by Yokota et al., Proc. Natl. Acad. Sci., Vol. 83, pgs. 5894-5898 (1986).

An activity designated BCGF II has also been described. It is characterized by a capacity to cause DNA synthesis in mitogen-stimulated B cells or in transformed B cell lines. Mitogens associated with BCGF II activity include dextran sulfate, lipopolysaccharide, and Staphylococcus extracts. BCGF I registers no response in these assays. In humans it is believed that BCGF II is a molecule having a molecular weight of about 50 kilodaltons (kD), and that it acts synergistically with BCGF I (i.e. BSF-1) in promoting B cell proliferation in an immune response: Yoshizaka et al., J. Immunol., Vol. 130, pgs. 1241-1246 (1983). In mice it is believed that BCGF II is a molecule having a molecular weight of about 55 kD by gel filtration chromatography and a mean isoelectric point of 5.5; Dutton et al., J. Immunol., Vol. 132, pg. 2451 (1984).

Recently it has been reported that BCGF II not only is a growth-inducing factor but also plays a role in polyclonal and antigen-specific B cell differentiation. Swain, J. Immunol., Vol. 134, pg. 3934 (1985), for example, showed that partially purified BCGF-II caused enhanced IgM secretion by $BCl_1$ lymphoma cells, whereas recombinant IL-2, recombinant gamma-interferon, and purified IL-4 had no such activity. Also, Takatsu et al., J. Immunol., Vol. 125, pg. 2646 (1980), and Ono et al., J. Immunol., Vol. 137, pg. 187 (1986) have identified a B cell differentiation factor (BCDF), designated B151-TRF1 (TRF for T-cell Replacing Factor), which was subsequently shown to have BCGF activity: Harada et al., J. Immunol., Vol. 134, pg. 3944 (1985), and Hara et al., Lymphokine Res., Vol. 4, pg. 243 (1985). The BCDF assay for B151-TRF1 measures IgG anti-DNP specific plaque-forming-cell (PFC)-responses of B cells derived from DNP-keyhole limpet hemagglutinin (KLH)-primed BALB/c mice and cocultured with supernatants containing B151-TRF1 and TNP-ovalbumin. BCGF II activity was assayed by induction of proliferation and immunoglobulin-secretion by $BCl_1$ lymphoma cells and by proliferation of dextransulfate-activated normal B cells.

Additional differentiation factor activity has been attributed to BCGF II. Sanderson et al., Proc. Natl. Acad. Sci., Vol. 83, pg. 437 (1986), and J. Exp. Med., Vol. 162, pg. 60 (1985), have identified an eosinophil differentiation factor (EDF) which appears additionally to display BCGF II activity. EDF was assayed by incubating bone marrow cells derived from Mesocestoides corti-infected mice with test supernatants, and measuring eosinophil development at 5 days by a colorimetric assay for eosinophil peroxidase. BCGF II activity was determined by the short-term $BCl_1$-proliferation assay referred to above. Gel filtration chromatography revealed an apparent molecular weight of 45-46 kD for both activities. Attempts to separate the activities by isoelectric focusing and/or lentil lectin, phenyl-Sepharose, DEAE-Sepharose, and reverse phase chromatography were unsuccessful. EDF was fractionated by SDS-PAGE in an attempt to separate the B cell growth promoting activity from the B cell differentiation activity. Both activities co-migrated in the same fractions corresponding to a molecular weight of 44 kD. EDF was reported to have a pI of 5.5. It is believed that eosinophils play a role in antiparasite immunity: Klein, Immunology (John Wiley & Sons, New York, 1982). At least one reported eosinophil differentiation factor appears to be pan-specific (rather than species-specific) among mammals (Metcalf et al., Blood, Vol. 61, pgs. 999-1005 (1983)), so that there may be significant homology between the factors of the various mammalian species.

There are many reports of B cell differentiation factors (BCDFs), that is, factors capable of inducing activated B cells to secrete IgM and/or IgG: e.g. Muraguchi et al., J. Immunol., Vol. 127, pg. 412 (1981), Yoshizaka et al., J. Immunol., Vol. 128, pg. 1296 (1982), and Teranishi et al., J. Immunol., Vol. 128, pg. 1903 (1982), to cite a few. A murine BCDF resembling human BCDF in terms of biological and biochemical properties has been identified in several laboratories: e.g. Pure et al., J. Immunol., Vol. 127, pg. 1953 (1981), and Nakanishi et al., J. Exp. Med., Vol. 160, pg. 1605 (1984). Biochemical characterization of this factor has revealed a molecular weight of 30-35 kD by gel filtration analysis and about 32 kD by SDS-PAGE. Other BCDFs, apparently distinct from that mentioned above, have also been reported, e.g. by Ono et al. (cited above), and Sidman et al., in J. Immunol., Vol. 132, pg. 209 (1984), and J. Immunol., Vol. 132, pg. 845 (1984). And still further BCDFs have been reported that have isotype-specific effects: e.g. Mayer et al., J. Exp. Med., Vol. 156, pgs. 1860-1865 (1982), report on IgA-specific BCDF.

The relationship of these various B cell factors to the production of specific immunoglobulin isotypes is presently not clear. However, better knowledge of the factors that determine which isotype is produced by a B cell could lead to significant new therapeutic approaches to infection. For example, IgA is the predominant immunoglobulin in the external secretions of humans. Specific IgA-producing cells home in on mucosal epithelia throughout the body, particularly the respiratory, urinogenital, and digestive tracts, to provide humoral protection as a frontline defense against pathogens borne in aerosols, the environment, and the diet. It is believed that an important function of IgA in these organs is to bind to invading microorganisms so as to reduce their mobility and their ability to adhere to the epithelial surface. IgA thereby renders the microorganisms susceptible to the natural cleansing functions of the mucosae. The ability to stimulate IgA secretion by administering an appropriate lymphokine could be an effective means for combatting a wide range of infectious disorders of the respiratory, urinogenital and gastrointestinal systems. It may also counteract natural inabilities to synthesize IgA, the most frequent immunoglobulin deficiency in humans, with a prevalence of 0.125-0.2 percent. Notably such deficiency is correlated with increased prevalence of childhood

**0 267 779**

infection, atopy, and circulating antibodies to food antigens: Underdown, Ann. Rev. Immunol., Vol. 4, pgs. 389-417 (1986).

In view of the foregoing, the development and/or discovery of factors which stimulate B cell growth and differentiation could lead to new approaches for treating diseases against which the humoral immune response is a primary body defense. In particular, the availability of an IgA enhancing factor could lead to new approaches for treating respiratory and gastrointestinal infections.

The present invention is addressed to problems associated with the application of immunoregulatory agents to treat medical and/or veterinary disorders. In particular, it provides compounds capable of stimulating the secretion of immunoglobulins by B lymphocytes and the development of eosinophils.

The present invention is directed to a new factor, human pleiotropic immune factor (PIF), and to novel muteins thereof. Human PIF includes polypeptides which comprise a sequence of amino acids selected from the set defined below by Formula I, and which possess B cell differentiation factor (BCDF) activity and eosinophil colony stimulating factor (Eo-CSF) activity as defined by standard assays. The invention also includes nucleic acids capable of encoding the polypeptides defined by Formula I, methods for making such polypeptides by use of the nucleic acids of the invention, and methods of using the polypeptides of the invention to treat disorders related to immunoglobulin deficiencies.

A preferred embodiment of the invention is the set of glycosylated or unglycosylated polypeptides which comprises a sequence of amino acids selected from the set of sequences defined by the following formula:

X(Glu) - X(Ile) - X(Pro) - X(Thr) - X(Ser) - X(Ala) -

10

X(Leu) - X(Val) - X(Lys) - X(Glu) - X(Thr) - X(Leu) -

X(Ala) - X(Leu) - X(Leu) - X(Ser) - X(Thr) - X(His) -

20

X(Arg) - X(Thr) - X(Leu) - X(Leu) - X(Ile) - X(Ala) -

30

X(Asn) - X(Glu) - X(Thr) - X(Leu) - X(Arg) - X(Ile) -

X(Pro) - X(Val) - X(Pro) - X(Val) - X(His) - X(Lys) -

40

X(Asn) - X(His) - X(Gln) - X(Leu) - X(Cys) - X(Thr) -

X(Glu) - X(Glu) - X(Ile) - X(Phe) - X(Gln) - X(Gly) -

50

X(Ile) - X(Gly) - X(Thr) - X(Leu) - X(Glu) - X(Ser) -

60

X(Gln) - X(Thr) - X(Val) - X(Gln) - X(Gly) - X(Gly) -

X(Thr) - X(Val) - X(Glu) - X(Arg) - X(Leu) - X(Phe) -

70

X(Lys) - X(Asn) - X(Leu) - X(Ser) - X(Leu) - X(Ile) -

X(Lys) - X(Lys) - X(Tyr) - X(Ile) - X(Asp) - X(Gly) -

4

```
                80
X(Gln) - X(Lys) - X(Lys) - X(Lys) - X(Cys) - X(Gly) -
                                              90
X(Glu) - X(Glu) - X(Arg) - X(Arg) - X(Arg) - X(Val) -

X(Asn) - X(Gln) - X(Phe) - X(Leu) - X(Asp) - X(Tyr) -
                100
X(Leu) - X(Gln) - X(Glu) - X(Phe) - X(Leu) - X(Gly) -

X(Val) - X(Met) - X(Asn) - X(Thr) - X(Glu) - X(Trp) -
        110
X(Ile) - X(Ile) - X(Glu) - X(Ser)
```

Formula I
wherein the terms X(Xaa) represent groups of synonymous amino acids and the numbers stand above the corresponding amino acids. Synonymous amino acids within a group have sufficiently similar physicochemical properties for substitution between members of the group substantially to preserve the biological function of the molecule: Grantham, Science vol. 185, pgs. 862-864 (1974). It is clear that insertions and deletions of amino acids may also be made in the above-defined sequence without altering biological function, particularly if only a few amino acids, e.g. under ten, are inserted or deleted, if amino acids that are critical to a functional conformation are not removed or replaced, e.g. cysteine residues: Anfinsen, "Principles That Govern The Folding of Protein Chains", Science, Vol. 181, pgs. 223-230 (1973). Proteins and muteins produced by such deletions and/or insertions come within the purview of the present invention. Whenever amino acid residues of the protein of Formula I are referred to herein by number, such a number is in reference to the N-terminus of the protein.

Preferably, the synonymous amino acid groups are those defined in Table I. More preferably, the synonymous amino acid groups are those underlined in Table I; and most preferably each synonymous amino acid group consists of a single amino acid, namely Xaa itself.

## Table I  Preferred Groups of Synonymous Amino Acids·

| Xaa | X(Xaa) |
|-----|--------|
| Ser | Ser, Cys |
| Arg | Arg, His, Lys |
| Leu | Leu, Ile, Met, Phe |
| Pro | Pro, Ala |
| Thr | Thr |
| Ala | Ala, Pro |
| Val | Val, Met, Ile |
| Gly | Gly |
| Ile | Ile, Met, Leu, Phe, Val |
| Phe | Phe, Met, Tyr, Ile, Leu |
| Tyr | Tyr, Phe |
| His | His, Gln, Arg |
| Gln | Gln, Glu, His |
| Asn | Asn, Asp |
| Lys | Lys, Arg |
| Asp | Asp, Asn |
| Glu | Glu, Gln |
| Met | Met, Ile, Leu, Phe, Val |
| Trp | Trp |
| Cys | Cys |

The invention includes the polypeptides of Formula I with amino acid substitutions (between an amino acid of the polypeptide defined by Formula II below (the putative native sequence) and a synonymous amino acid) at a single position or at multiple positions. The term "N-fold substituted" is used to describe a subset of polypeptides defined by Formula I wherein from 0-N synonymous amino acids have been substituted for from 0-N amino acids of the native sequence. Thus, for example, the group of 1-fold substituted polypeptides of Formula I consists of 182 polypeptides for the preferred groups of synonymous amino acids, and 50 for the more preferred groups of synonymous amino acids.

Preferably, the group of human PIF polypeptides consists of the 10-fold substituted polypeptides of Formula I; more preferably, the group of human PIF polypeptides consists of the 5-fold substituted polypeptides of Formula I; still more preferably, the group of human PIF polypeptides consists of the 1-fold substituted polypeptides of Formula I. Most preferably, the polypeptides of the invention comprise the native amino acid sequence defined by the following formula:

```
Glu - Ile - Pro - Thr - Ser - Ala - Leu - Val - Lys -
10
Glu - Thr - Leu - Ala - Leu - Leu - Ser - Thr - His -
    20
Arg - Thr - Leu - Leu - Ile - Ala - Asn - Glu - Thr -
        30
Leu - Arg - Ile - Pro - Val - Pro - Val - His - Lys -
        40
Asn - His - Gln - Leu - Cys - Thr - Glu - Glu - Ile -
        50
Phe - Gln - Gly - Ile - Gly - Thr - Leu - Glu - Ser -
            60
Gln - Thr - Val - Gln - Gly - Gly - Thr - Val - Glu -
            70
Arg - Leu - Phe - Lys - Asn - Leu - Ser - Leu - Ile -

            80
Lys - Lys - Tyr - Ile - Asp - Gly - Gln - Lys - Lys -
                90
Lys - Cys - Gly - Glu - Glu - Arg - Arg - Arg - Val -
Asn - Gln - Phe - Leu - Asp - Tyr - Leu - Gln - Glu -
100
Phe - Leu - Gly - Val - Met - Asn - Thr - Glu - Trp -
    110
Ile - Ile - Glu - Ser.
```

Formula II

Likewise, the term "N-fold inserted" in reference to the polypeptides of Formula I is used to describe a set of polypeptides wherein from 1 to N amino acids have been inserted into the sequence defined by Formula I. Preferably, the inserted amino acids are selected from the preferred groups of synonymous amino acids (Table I) of either amino acid flanking the insertion; more preferably they are selected from the more preferred groups of synonymous amino acids (Table I) of either amino acid flanking the insertion. Thus, for example, one subgroup of the group of 1-fold inserted peptides comprises an amino acid inserted between the N-terminal X(Glu) and the adjacent X(Ile). The inserted members of this subgroup are preferably selected from Glu, Gln, Met, Phe, Ile, Val, and Leu; more preferably they are selected from Met, Leu, Ile, and Glu, and most preferably they are selected from Ile and Glu. Insertions can be made between any adjacent amino acids of Formula I. Since there are 111 possible insertion locations, and since multiple insertions can be made at the same location, a 2-fold inserted peptide of Formula I gives rise to $111 \times 111 = 12{,}321$ subgroups of peptides, and the size of each subgroup depends on the sizes of the synonymous amino acid groups of the amino acids flanking the insertions.

The term "N-fold deleted" in reference to the polypeptides of Formula I is used to describe a set of peptides having from 1 to N amino acids deleted from the sequence defined by Formula I. Thus, the set of 1-fold deleted polypeptides of Formula I consists of 112 subgroups of polypeptides each 111 amino acids in length (111-mers). Each of the subgroups in turn consists of all the 111-mers defined by the preferred, more preferred, and most preferred synonymous amino acid groups depending on the multiplicity of substitutions.

Whenever a set of polypeptides is defined in terms of both deletions with respect to the sequence of Formula I and insertions with respect to the sequence of Formula I, first the set of polypeptides defined by the insertions is formed and then for each member of that set a set of polypeptides defined by deletions is formed. The sum of the members of all the latter sets forms the set of polypeptides defined by both insertions and deletions.

The invention further includes nucleotide sequences capable of encoding the polypeptides of Formula I and nucleic acid cassettes capable of encoding portions of the polypeptides of Formula I for the preferred, more preferred, and most preferred groups of synonymous amino acids. As used herein, "nucleic acid cassette" means a nucleic acid of 10-120 bases (i) which has a nucleotide sequence capable of encoding a portion of a polypeptide defined by Formula I, and (ii) whose ends are defined by restriction endonuclease sites which are unique with respect to the cloning or expression vector containing the nucleic acid and its complementary strand. As explained more fully below, nucleic acid cassettes are used to construct mutant forms of PIF.

Throughout, standard abbreviations are used to designate amino acids, nucleotides, restriction endonucleases, and the like: e.g. Cohn, "Nomenclature and Symbolism of α-Amino Acids", Methods in Enzymology, Vol. 106, pgs. 3-17 (1984); Wood et al., Biochemistry: A Problems Approach, 2nd ed. (Benjamin, Menlo Park, 1981); and Roberts, "Directory of Restriction Endonucleases", Methods in Enzymology, Vol. 68, pgs. 27-40 (1979).

For the better understanding of the invention, preferred embodiments thereof will be described with reference to the accompanying drawings wherein:

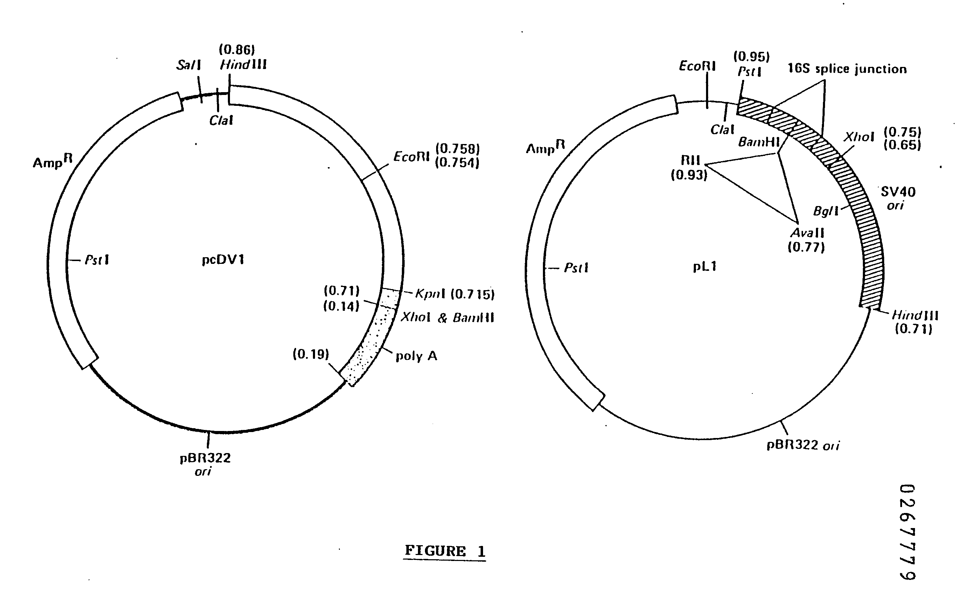

Figure 1 diagrammatically illustrates restriction sites and major coding regions of plasmids pL1 and pcDV1 used in constructing the pcD expression and cloning vector; and

Figure 2 illustrates the nucleotide sequence and predicted amino acid sequence of a mouse PIF.

The present invention includes human pleiotropic immune factors which exhibit Eo-CSF activity and BCDF activity. The factors either are produced directly from a pool of pcD expression vectors designated 114 by standard transfection techniques followed by biochemical purification, or are derivable from the cDNAs of pool 114 first by isolation of a single cDNA clone which encodes the above activities, followed either by propagation and expression of the isolated cDNA, or by modification of the isolated cDNA to produce muteins, or by use of the isolated cDNA or its mutants to probe genomic or cDNA libraries of other mammalian species for homologous cDNAs capable of encoding additional factors of the invention.

Pool 114 has been deposited with the American Type Culture Collection (ATCC) under accession number 67267 and contains 537 ± 23 cDNA clones, at least one of which encodes the pleiotropic immune factor of the invention.

Techniques for making, using, and identifying the polypeptides and nucleic acids of the invention are discussed below in general terms. Afterward several specific examples are provided wherein the general techniques are applied using specific cell types, vectors, reagents, and the like.

## I. De Novo Preparation of PIF cDNA

A variety of methods are now available for de novo preparation and cloning of cDNAs, and for the construction of cDNA libraries; e.g. recent reviews are given by Doherty, "Cloning and Expression of cDNA", Chapter 10 in Gottesman, Ed., Molecular Cell Genetics (John Wiley & Sons, New York, 1985), and Brandis et al., "Preparation of cDNA Libraries and the Detection of Specific Gene Sequences", in Setlow et al., Eds. Genetic Engineering, Vol. 8, pgs. 299-316 (Plenum Press, New York, 1986).

By way of example, total mRNA is extracted (e.g., as reported by Berger, S. et al., Biochemistry 18 5143-5149 [1979]) from cells (e.g., a nontransformed human T cell source) producing polypeptides exhibiting the desired activity. The double-stranded cDNAs from this total mRNA can be constructed by using primer-initiated reverse transcription (Verme, I., Biochem. Biophys. Acta, Vol. 473, pgs. 1-38 [1977]) to make first the complement of each mRNA sequence, and then by priming for second strand-synthesis (Land, H. et al., Nucleic Acids Res., 9: 2251-2266 [1981]). Subsequently, the cDNAs can be cloned by joining them to suitable plasmid or bacteriophage vectors (Rougeon, F. et al., Nucleic Acids Res., 2, 2365-2378 [1975]) or Scherer, G. et al., Dev. Biol. 86, 438-447 [1981]) through complementary homopolymeric tails (Efstratiadis, A. et al., Cell, 10, 571-585 [1977]) or cohesive ends created with linker segments containing appropriate restriction sites (Maniatis et al., Molecular Cloning: A Laboratory Manual, Cold Spring Harbor Laboratory, N.Y. 1982), and then transforming a suitable host. (See generally Efstratiadis, A., and Villa-Kormaroff, L., "Cloning of double stranded cDNA" in Setlow, J. and Hollaender, A. (eds.) Genetic Engineering, Vol. 1, Plenum Publishing Corp., N.Y., U.S.A. [1982].)

A preferred source of mRNA encoding the desired polypeptides is cells whose supernatants contain Eo-CSF and/or IgA-Enhancing Factor (IgA-EF) activities, or other activities associated with the polypeptides of the present invention. One such line is the mouse T cell line Cl.Lyl+2−/9 (ATCC Accession No. CRL8179) (Nabel, G. et al., Nature, 291:332-334 (1981)). In general, suitable T cells can be obtained from a variety of sources, such as mammalian (e.g. human) spleen, tonsils and peripheral blood. T cell clones, such as those isolated from peripheral blood T-lymphocytes, may also be used (see Research Monographs in Immunology, eds. von Doehmer, H. and Haaf, V.; Section D: "Human T Cell Clones", vol.8, pgs. 243-333; Elsevier Science Publishers, N.Y. [1985]).

Production of mRNAs capable of coding for PIF by such cells can be confirmed by microinjection of the extracted mRNA into oocytes of Xenopus laevis. This microinjection technique is described more fully below, and is disclosed generally in Colman et al., "Export of Proteins from Oocytes of Xenopus laevis", Cell Vol. 17, pgs. 517-526 (1979); and Maniatis et al. Molecular Cloning: A Laboratory Manual, pgs. 350-352 (Cold Spring Harbor Laboratory, New York, 1982).

If the mRNAs coding for a desired PIF make up a very small fraction of the total mRNA, it may be necessary to enrich the fractional concentration in order to make practical the screening procedure for detecting cDNA clones of interest. Such procedures are standard in the art and are disclosed in several papers and references, such as Maniatis et al., pgs. 225-228, cited above; Suggs et al., Proc Natl. Acad. Sci., Vol. 78, pgs. 6613-6617 (1981); Parnes et al., Proc. Natl. Acad. Sci., Vol. 78, pgs. 2253-2257 (1981), Davis et al., Proc. Natl. Acad. Sci., Vol. 81, pgs. 2194-2198 (1984) or the like.

A preferred method of de novo preparation of PIF cDNAs relies on functional expression of the cDNAs in the pcD expression system developed by Okayama and Berg ("functional cloning"), disclosed in Mol. Cell. Biol., Vol. 2, pgs. 161-170 (1982); and Vol. 3. pgs. 280-289 (1983), and available from Pharmacia (Piscataway, N.J.). The pcD expression vector contains the SV40 early promoter, late splicing junction, and the replication origin. This vector permits expression of cDNA inserts in COS 7 monkey cells which provide T antigen for replication of the pcD plasmid. Screening of cDNA libraries includes transfection of pools of plasmid DNA into COS 7 cells using DEAE-Dextran. Since lymphokines, and in particular PIFs, are secreted proteins, the supernatants from the transfected cells can be assayed for biological activity after incubation for several days. Positive pools are further divided to identify single cDNA clones which give biological activity after transfection.

Briefly, the Okayama and Berg expression vector is constructed as follows. Polyadenylated mRNA is annealed to a polydeoxythymidylic acid (oligo dT) tail attached to the protruding strand of a KpnI-digested pBR322 plasmid containing the SV40 early promoter region. That is, the entire vector serves as a primer for cDNA synthesis. After cDNA synthesis, 3'-polydeoxycytidylate (oligo dC) tails are attached, and the DNA product is digested with HindIII, which lops off (at a unique Hind III site) a fragment of the SV40 DNA to which one of the oligo dC tails is attached. The SV40 early promoter remains intact, and fortuitously occurring HindIII sites of the insert are affected minimally because the hybrid cDNA/RNA is resistant to HindIII digestion. A separately constructed HindIII fragment having a 3'-polyguanidylated (oligo dG) tail is annealed to the sticky end left by the HindIII digestion. The vector is circularized and treated with E. coli RNase H, DNA polymerase I, and DNA ligase to replace the RNA strand with DNA. The vectors are cloned in E. coli to form the cDNA library. The SV40 elements permit the vectors to be expressed in eucaryotic cells as well as procaryotic cells, and particularly in mammalian cells, such as COS7 monkey cells or Chinese hamster ovary (CHO) cells.

Once the cDNA library in the Okayama/Berg plasmid vector has been completed, the cDNA clones are collected, and random pools checked for the presence of the desired cDNAs by standard procedures, e.g. hybrid selection, detection of antigenic determinants on expressed products, and/or functional assays. Positive pools can then be probed with a cDNA from an induced T cell line. Thereafter, the positive, probed pools are divided into individual clones which are further tested by transfection into a suitable host (such as mammalian cells in culture), and the host supernatant is assayed for activity.

## II. Preparation of PIF cDNAs Via Hybridization Probes Derived from Disclosed cDNAs

The cDNAs disclosed herein can be used as probes to identify homologous sequences in different cell types, as an alternative to de novo isolation and cloning of the PIF-coding nucleotides. Standard techniques are employed, e.g. Callahan et al., "Detection and Cloning of Human DNA Sequences Related to the Mouse Mammary Tumor Virus Genome," Proc. Natl. Acad. Sci., Vol. 79, pgs. 5503-5507 (1982); and especially Beltz et al., "Isolation of Multigene Families and Determination of Homologies by Filter Hybridization Methods," Methods in Enzymology, Vol. 100, pgs. 266-285 (1983). Basically, the cDNAs of the invention are used to construct probes (using standard techniques, e.g. see Maniatis et al., cited above) for screening at low hybridization stringencies genomic or cDNA libraries (again, constructed by standard techniques) of a cell type suspected of producing PIF. Standard screening procedures are employed, e.g. Grunstein et al., Proc. Natl. Acad. Sci., Vol. 72, pgs. 3961-3965 (1975); or Benton et al., Science Vol. 196, pgs. 180-183 (1977).

## III. Preparation of PIF Muteins by Protein Engineering

Once nucleic acid sequence and/or amino acid sequence information is available for a native protein, a variety of techniques become available for producing virtually any mutation in the native sequence. Shortle, in Science, Vol. 229, pgs. 1193-1201 (1985), reviews techniques for mutating nucleic acids which are applicable to the present invention. Preferably, mutants of the native PIFs, i.e. PIF muteins, are produced by site-specific oligonucleotide-directed mutagenesis; see in particular Zoller and Smith, Methods in Enzymology, Vol. 100, pgs. 468-500 (1983), and Mark et al., U.S. Patent 4,518,584 entitled "Human Recombinant Interleukin2 Muteins"; or by so-called "cassette" mutagenesis described by Wells et al. in Gene Vol. 34, pgs. 315-323 (1985); and Estell et al., Science Vol. 233, pgs. 659663 (1986); and also described essentially by Mullenbach et al., J. Biol. Chem., Vol. 261, pgs. 719-722 (1986), and Feretti et al., Proc. Natl. Acad. Sci., Vol. 83, pgs. 597-603 (1986).

In sections below the notation used by Estell et al. (cited above) to identify muteins is followed and generalized. For example, "human PIF mutein Leu$^2$" (or simply "Leu$^2$" if the native protein is understood from the context) indicates a polypeptide whose amino acid sequence is identical to that of the native protein except for position 2 with respect to the N-terminus; at that position Leu has been substituted for Ile. Similarly, a mutein containing more than one substitution, namely Leu for Val at position 2 and Ile for Leu at position 12, is referred to as human PIF mutein (Leu[2], Ile[12]). Deletions are indicated by "$\Delta$'s". For example, a mutein lacking Thr at position 4 is referred to as human PIF mutein $\Delta 4$. Insertions are indicated by "IN(Xaa)'s". For example, a mutein with a Leu inserted after Ala at position 6 is referred to as human PIF mutein IN[6](Leu). Thus, human PIF mutein (Ser[11], $\Delta 4$, IN[6](Leu)) represents the native human PIF sequence which has been modified by replacing Thr by Ser at position 11, deleting Thr at position 4, and inserting Leu immediately after Ala at position 6. Insertion of multiple amino acids at the same site is indicated by $IN^i(Xaa_1\text{-}Xaa_2\text{-}Xaa_3\text{-}...)$, where $Xaa_1\text{-}Xaa_2\text{-}Xaa_3...$ is the sequence inserted after position i. N-terminal additions are indicated by superscript "0", e.g. IN[0](Xaa), and a sequence of deletions, for example of amino acids 6-10, is designated either as $\Delta^{6-10}$ or as $(\Delta^6, \Delta^7, \Delta^8, \Delta^9, \Delta^{10})$.

Most preferably cassette mutagenesis is employed to generate human PIF muteins. As described more fully below, a synthetic human PIF gene is constructed with a sequence of unique restriction endonuclease sites spaced approximately uniformly along the gene. The word "unique" indicates that these restriction sites remain unique when the gene is inserted into an appropriate vector. The unique restriction sites allow segments of the gene to be conveniently excised and replaced with synthetic oligonucleotides (i.e. "cassettes") which code for desired muteins.

Determination of the number and distribution of unique restriction sites entails the consideration of several factors including (1) preexisting restriction sites in the vector to be employed in expression, (2) desirability of species- or genera-specific codon usage, (3) which restriction endonucleases do not cleave the vector, and how many restriction sites they have within the synthetic gene, and (4) the convenience and reliability of synthesizing and/or sequencing the segments between the unique restriction sites.

Generally, Formula I defines sets of polypeptides with conservative amino acid substitutions, insertions, and/or deletions with respect to the putative native human PIF sequence. "Conservative" as used herein means (i) that the alterations are as conformationally neutral as possible, that is, designed to produce minimal changes in the tertiary structure of the mutant polypeptides as compared to the native human PIF, and (ii) that the alterations are as antigenically neutral as possible, that is, designed to produce minimal changes in the antigenic determinants of the mutant polypeptides as compared to the native human PIF. Conformational neutrality is desirable for preserving biological activity, and antigenic neutrality is desirable for avoiding the triggering of immunogenic responses in patients.

Whereas it is difficult to select with absolute certainty which alternative amino acid residues will be conformationally and antigenically neutral, rules exist which can guide those skilled in the art to make alterations that have high probabilities of being conformationally and antigenically neutral; e.g. Anfisen (cited above), and Berzofsky, Science, Vol. 229, pgs. 932-940 (1985). Some of the more important rules are: (1) replacement of hydrophobic residues is less likely to produce changes in antigenicity because they are likely to be located in the protein's interior (e.g. Berzofsky, cited above); (2) replacement of physiochemically similar, i.e. synonymous, residues is less likely to produce conformational changes because the replacing amino acid can play the same structural role as the displaced amino acid; and (3) alteration of evolutionarily-conserved sequences is likely to produce deleterious conformational effects because evolutionary conservation suggests that sequences may be functionally important.

In addition to such basic rules for selecting mutein sequences, assays are available to confirm the biological activity and conformation of the engineered molecules. Biological assays for the polypeptides of the invention are described more fully below. Changes in conformation can be tested by at least two well-known assays: the microcomplement fixation method, e.g. Wasserman et al., J. Immunol., Vol. 87, pgs. 290-295 (1961), or Levine et al. Methods in Enzymology, Vol. 11, pgs. 928-936 (1967), used widely in evolutionary studies of the tertiary structures of proteins; and affinities to sets of conformation-specific monoclonal antibodies, e.g. Lewis et al., Biochemistry, Vol. 22, pgs. 948-954 (1983).

IV. Assays for Biological Activity.

A. IgA-Enhancing Factor Activity.

IgA-EF activity can be assayed by a standard isotype-specific enzyme-linked immunosorbent assay (ELISA) technique which quantitatively measures the amounts of the various isotypes produced by a population of purified B cells that have been stimulated to proliferate and differentiate into immunoglobulin-secreting cells. Isotype enhancement is determined by comparing the relative amounts of a given isotype, e.g. IgM, produced by the stimulated B cells in the presence and in the absence of the suspected enhancing agent. Coffman and Carty, J. Immunol., Vol. 136, pgs. 949-954 (1986) disclose an important ELISA assay designed for such measurements. This assay requires the use of isotype-specific antibodies, which are available commercially or can be obtained by standard techniques as described below. The assay also requires a population of purified B cells, which is obtained using standard techniques, e.g. Coffman and Carty (cited above). Preferably the B cells are from the same species as the PIF being assayed.

Isotypic antisera are obtained by immunizing a host animal, e.g. rabbit, with a monoclonal of the desired isotype in a suitable adjuvant, e.g. Freund's. Preferably, the antisera are rendered heavy-chain-specific by passage through affinity columns of Sepharose (Pharmacia, Piscataway, N.J.) or Affi-gel (Bio-Rad, Richmond, CA) conjugated with immunoglobulins of the undesired isotypes. Anti-isotypic antibodies from the antisera are then bound and next eluted from an affinity column conjugated with a monoclonal immunoglobulin of the desired isotype. Preferably, this latter monoclonal has a binding specificity different from that of the monoclonal used to immunize the host animal. This avoids contamination of isotype-specific antibodies with idiotype-specific antibodies.

The isotype-specific antibodies are derivatized for use as second-step reagents in the isotype-specific ELISA by standard techniques, e.g. Tijssen, Practice and Theory of Enzyme Immunoassays (Elsevier, New York, 1985). For example, antibodies may be derivatized with NIP (nitroiodo-phenyl)-succinimide ester, biotin succinimide ester (Bioresearch, San Rafael, CA), or similar reagents.

Preferably, purified B cell populations are obtained by selectively killing non-B cells with monoclonal antibodies specific for surface markers on the non-B cells. Sources of B cells include peripheral blood, spleen, and tonsils.

T cell-depleted spleen cell suspensions from mice are prepared by forcing spleen fragments through a 200-mesh wire screen. The cell suspension is then washed and incubated for 15 minutes on ice with anti-Thy-1.2 (e.g. produced by hybridoma 30-H12 deposited with the ATCC under accession number TIB 107). The cells are then pelleted by centrifugation, and resuspended in 10% rabbit complement (Lo-Tox M, Cedarlane Laboratories, Hornby, Ontario, Canada), diluted in RPMI 1640 medium containing 25 mM HEPES, pH 7.2, and 0.3% bovine serum albumin. Cells are incubated with complement for 45 minutes at 37°C. Dead cells are removed by centrifugation over Ficoll (density 1.119 g/ml, Sigma Chemical Co., St. Louis, MO).

Alternatively, purified populations of B cells can be prepared by incubating with a mixture of monoclonal antibodies, for example to Thy-1, L3T4 (e.g. from hybridoma GK1.5; ATCC accession number TIB 207), Lyt-2 (e.g. from hybridoma 53-6.72, ATCC accession number TIB 105), MAC-1 (e.g. from hybridoma MI/70; ATCC accession number TIB 128), or the like, followed by complement treatment and a final centrifugation as described above for the removal of dead cells.

Enriched populations of human B cells for the assay can be produced by repeated rosetting with 2-aminoethyl-isothiouronium bromide-treated sheep erythrocytes to eliminate T cells; e.g. Saxon et al., J. Immunol. Meth., Vol. 12, pg. 285 (1976). Rosettes are then removed on Ficoll-Hypaque gradients as disclosed by Saxon et al. (cited above). Purity of the B cell population can be assayed by determining the fractions of cells positive for B cell-specific and T cell-specific antigens, e.g. by using labeled monoclonal antibodies such as B1 (B cell specific) from Coulter (Hialeah, FL), Leu-1 (T cell specific) from Becton-Dickinson (Mountain View, CA), OKT 11 (T cell specific) from Ortho Diagnostics (Westwood, MA), or the like. Preferably, T cell contamination should be less than 1% by these assays. Alternatively, T cells can be removed by treating with anti-Leu-1 and rabbit complement, e.g. as described by Falkoff et al. J. Immunol. Meth., Vol. 50, pg. 39 (1982).

The purified B cells are stimulated with an agent or combination of agents (referred to herein collectively as a stimulatory agent) which causes the B cells to become immunoglobulin-secreting cells. (B cells exposed to such a stimulatory agent are referred to herein as stimulated B cells.) Isotype-enhancing or -suppressing activities of a compound are determined by comparing the relative levels of the various isotypes produced by a control population of stimulated B cells and by a population of stimulated B cells exposed to the compound. For mouse B cells the preferred stimulatory agent is lipopolysaccharide (LPS) (e.g. Salmonella typhimurium LPS available from Sigma). For human B cells the preferred stimulatory agent is Staphylococcus aureus Cowan I (SAC) (e.g., available in a 0.01% solution from Calbiochem under the tradename Pansorbin, or preparable as described by Falkoff et al., J. Immunol., Vol. 129, pgs. 97-102 (1982)) plus a standardized quantity of a B cell differentiating factor (BCDF). BCDF can be provided from culture supernatant of mitogen-stimulated helper T cells, e.g. isolated from the peripheral blood, or from culture supernatant of constitutively producing hybridomas, such as those described by Mayer et al. J. Exp. Med., Vol. 156, pgs. 1860-1865 (1982).

Purified mouse B cells are cultured in round-bottomed, 96-well plates (e.g. available from Flow Laboratories, McClean VA), or like containers, in RPMI 1640 medium (GIBCO) plus penicillin, streptomycin, glutamine, 2-mercaptoethanol ($5 \times 10^{-5}$ M), 10% fetal calf serum (e.g. Hyclone, Logan, UT), and about 4 micrograms/ml LPS, at a concentration of about $0.5-1.0 \times 10^6$ cells/ml. Purified human B cells are cultured in similar containers in Yssel's medium (Yssel et al., J. Immunol. Meth., Vol. 65, pgs. 55-63 (1984)) with 0.01% SAC and a standardized quantity of BCDF at about $5 \times 10^5$ cells/ml. In either case, after one day, dilutions of the sample to be assayed are added to the culture plates, e.g. in 0.1 ml aliquots, and the plates are incubated for 5-7 days, after which the culture supernatants are harvested for ELISA.

Isotype-specific ELISA is carried out as follows. Polyvinyl chloride 96-well plates (Dynatech, Alexandria, VA) are coated for 1 hr with the appropriate first step isotype-specific antibody at concentrations of 0.5 to 2.0 µg/ml. These plates are blocked with phosphate-buffered saline plus 0.1% bovine serum albumin and 0.04% Tween 20. Standard curve solutions and supernatants to be tested are added in 0.1 ml, all dilutions being made in RPMI 1640 plus 5% fetal calf serum. After 3 hr at room temperature, the plates are washed once in phosphate-buffered saline plus 0.04% Tween 20 and the appropriate NIP- or biotin-conjugated second step antibody at concentrations of 0.25 to 2.0 µg/ml. (NIP is 4-hydroxy-3-iodo-5-nitrophenyl acetic acid.) After incubation for 1 hr at room temperature, these plates are washed as before, and an optimum concentration of either horseradish peroxidase-conjugated monoclonal anti-NIP or horseradish peroxidase-conjugated avidin (Vector Laboratories, Burlingame, CA) are added. One hour later the plates are washed, and 0.1 ml is added of a substrate solution containing 1 mg/ml 2,2'-azinobis(3-ethyl-benzothiazolinesulfonic acid) (Sigma) and 0.003% $H_2O_2$ in 0.1 M $Na_2HPO_4$ and 0.05 M citric acid. The reaction is stopped by the addition of 0.05 ml of 0.2 M citric acid, and the plates are read on a Dynatech ELISA reader, or the like.

11

B. BCDF Activity

BCDF activity is assayed by measuring the extent to which purified proliferating B cells are induced to secrete immunoglobulins. Some forms of the assay are similar to the IgA-EF assay, except that instead of exposing the purified B cells to a stimulatory agent (as defined above) the purified B cells are exposed to an agent which induces them to start progressing through the cell cycle, but not to differentiate (i.e. to secrete immunoglobulin), e.g. Butler et al., Proc. Natl. Acad. Sci., Vol. 81, pgs. 2475-2478 (1984). Such agents are referred to herein as B cell mitogens. Secreted antibodies can be determined by ELISA, or by reverse hemolytic plaque assay, e.g. as disclosed by Gronowicz et al., Eur. J. Immunol., Vol. 6, pg. 588 (1986). For human cells, B cell mitogens include SAC (at about 0.01-0.001% (v/v) concentration), $F(ab')_2$ fragments of goat antihuman IgM chain (e.g. available from Cappel Laboratories, Cochranville, PA), and the like. Actual concentrations used may have to be adjusted for the particular experimental system employed. Other BCDF assays do not require the use of a B cell mitogen. For example, the following protocol for assaying murine BCDF does not require the use of B cell mitogens. Materials used in the protocol are as follows: Cytotoxicity medium (CTM) is diluted from a 10 × stock solution containing 3.0g fraction V bovine serum albumin (BSA) and 6.0g HEPES (both available from Sigma) in 100 ml total volume of RPMI medium (see ATCC Catalogue of Cell Lines & Hybridomas, 5th ed. (1985) for a description of and bibliography for RPMI media). The pH is adjusted to 7.2 with 10 N NaOH prior to final dilution. CTM and all other solutions described below contain penicillin (50 U/ml), streptomycin (50 mgc/ml), and gentamicin sulfate (5 micrograms/ml), and are sterilized by passage through a 0.22 micrometer membrane. MD medium is 10% fetal bovine serum (FBS) (e.g. from Hyclone), 50 micromolar beta-mercaptoethanol, 1.0mM glutamine, and 1.0 mM sodium pyruvate in RPMI. Preferably all steps of the protocol are carried out at, or close to, 4°C under sterile conditions. Centrifugation steps are at 250g for 7 minutes. B cells are obtained from spleens, e.g. from C3H/HeJ male mice, preferably between 6 and 8 weeks old.

B cells are purified as follows. A spleen cell suspension is obtained by the crushing of the spleens between two sintered glass slides. Between 35 and 45 ml of RPMI with 10% FBS is used to suspend cells from 6 to 12 spleens. These suspended cells are collected by centrifugation. The resultant pellet is resuspended in 40 ml of 170 mM $NH_4Cl$, 4% FBS to lyse red blood cells present. The resulting suspension is immediately centrifuged through a cushion of 2.0 ml of FBS. The pellet is washed with RPMI, 10% FBS with care taken to discard non-resuspendable cellular debris. The total number of cells present is determined by the counting of an aliquot of a known dilution of the spleen cell suspension using a hemocytometer. The above washed pellet is resuspended in a volume of cytotoxicity medium (CTM) calculated to yield a concentration of $1.0 \times 10^7$ cells ml$^{-1}$.

For selective T cell lysis by rabbit complement, to the above spleen cell suspension in CTM are added monoclonal antibodies directed against the T cell marker Thy1.2 and the T helper cell marker L3T4. The anti-Thy1.2 and anti-L3T4 are furnished by the cell lines 30H12 and GK1.5, respectively. The amount of monoclonal antibody used is based on the ability of either the 30H12- or GK1.5-produced antibody to yield maximal spleen cell killing upon subsequent treatment with rabbit complement as described for the T cell lysis step below. After incubation of the spleen cell-monoclonal antibody mixture for 60 min. on ice, cells are centrifuged and washed once with CTM.

To each vial of rabbit complement used is added in 1.0 ml of sterile mill-Q $H_2O$ to resuspend its contents. Vials used are then pooled and diluted with an equal volume of CTM and then filtered through coupled 0.45 and 0.22 μm filters which are Millex-HA and Millex-GV, respectively. The filtered rabbit complement is then diluted five-fold with CTM to yield a net dilution of ten-fold. This solution is used to resuspend the above, once washed, cell pellet to a density of $1.0 \times 10^7$ cells ml$^{-1}$. After the antibody-incubated cells are resuspended with the rabbit-complement-containing CTM, lysis is allowed to occur for a period of 60 min. at 37°C.

To remove non-viable cells after the lysis step, the cell suspension is centrifuged, washed once with CTM, and resuspended in CTM to a density of $2.5 \times 10^7$ cells ml$^{-1}$; then visible cellular debris that cannot be resuspended is discarded after the first centrifugation step. Aliquots of 4.0 ml of this suspension are gently layered each onto 4.0 ml of Histopaque 1119 (Ficoll type 400 from Sigma) to form similar but separate discontinuous gradients (one for each aliquot). The polystyrene tubes which contain the Histopaque are centrifuged at 1100 × g for a period of 20 min. at room temperature with the brake turned off. The interface is harvested from each ficoll gradient, separately diluted to 50 ml with CTM, and centrifuged. The cell pellets are combined and washed twice with MD medium. Finally, the T-cell-depleted spleen cells are diluted to a density between $1.1 \times 10^5$ and $1.5 \times 10^5$ cells ml$^{-1}$ in MD medium. The resultant yield is usually between 10% and 20% of the input cells. Of this final suspension, 90 μl is added to the wells of Costar A/2 96-well tissue culture plates. Cells are cultured overnight at 37°C in a 5% $CO_2$ incubator before adding test samples.

Test samples are added in volumes of from 1.0 to 5.0 microliters and the cultures are incubated as described above for 3 to 4 days, after which culture supernatants are harvested, diluted 100-220-fold in RPMI with 5% FBS, and tested for secreted immunoglobulin by ELISA as described above.

An alternative BCDF assay for human factors measures increase in IgG production in the human lymphoblast cell line CESS, which is available from the ATCC under accession number TIB 190 and is described in Brit. J. Haematol., Vol. 51, pgs. 595-604 (1982), and J. Immunol., Vol. 127, pg. 412 (1981).

## C. Eo-CSF Activity.

Eosinophil growth and development from hemopoietic precursor cells is measured by standard colony stimulating factor (CSF) assays. That is, the precursor cells are placed in standard in vitro cultures containing the compound suspected of having CSF activity. After allowing time for colony growth and development, the cultures are examined to determine the number of colonies formed, the size of colonies formed, and the types of cells present in the colonies, e.g. macrophages, megakaryocytes, eosinophils, etc. Well-defined criteria are used to identify the various cell types. For example, after glutaraldehyde fixation eosinophils are specifically stained with Luxol Fast Blue.

To determine CSF activity, hemopoietic cells, e.g. bone marrow cells or fetal-cord blood cells, are made into a single cell suspension. The individual cells are then "immobilized" in a semi-solid (agar) or viscous (methylcellulose) medium containing nutrients and usually fetal calf serum. In the presence of an appropriate stimulating factor (e.g. contained in a test sample), individual cells will proliferate and differentiate. Since the initial cells are immobilized, colonies develop as the cells proliferate and mature. These colonies can be scored after 7-14 days: Burgess, A., Growth Factors and Stem Cells, pgs. 52-55, Academic Press, New York [1984]. (For specific application to the growth of granulocytes and macrophages, see Bradely, T. and Metcalf, D., Aust. J. Exp. Biol. Med. Sci., Vol. 44, pgs. 287-300 [1966], and see generally Metcalf, D., Hemopoietic Colonies, Springer-Verlag, Berlin [1977].) If desired, individual colonies can be extracted, placed on microscope slides, fixed and stained with Wright/Geimsa (Todd-Sanford, Clinical Diagnosis by Laboratory Methods, 15th Edition, Eds. Davidson and Henry [1974]). Morphological analysis of cell types present per single colony can then be determined.

Bone marrow cells collected from patients with nonhematologic disease are layered over Ficoll (type 400, Sigma Chemical Co., St. Louis, MO) and centrifuged (600 $\times$ g), and the cells at the interface are removed. These cells are washed twice in Iscove's Modified Dulbecco's Medium containing 10% fetal calf serum (FCS) and resuspended in the same medium, and the adherent cells are removed by adherence to plastic Petri dishes. The nonadherent cells are added at $10^5$ cells/ml to Iscove's Medium containing 20% FCS, 50 $\mu$M 2-mercaptoethanol, 0.9% methylcellulose and various concentrations of either supernatants known to contain colony stimulating activity or test supernatants. One ml aliquots are plated in 35 mm petri dishes and cultured at 37°C in a fully humidified atmosphere of 6% $CO_2$ in air. Three days after the initiation of the culture, 1 unit of erythropoietin is added to each plate. Granulocyte-macrophage colonies and erythroid bursts are scored at 10-14 days using an inverted microscope.

Cord blood cells collected in heparin are spun at 600 $\times$ g. The white blood cells at the interface between the plasma and red blood cell peak are transferred to a tube containing 0.17 N ammonium chloride and 6% FCS. After 5 min on ice, the suspension is underlaid with 4 ml FCS and centrifuged at 600 $\times$ g. The cell pellet is washed with Dulbecco's-phosphate buffered saline and put through the Ficoll and plastic adherence steps as described above for bone marrow cells. The low- density nonadherent cells are collected and placed at $10^5$ cells/culture in the semi-solid culture medium as described above.

At the end of the assays, the cellular composition is determined after applying the individual colonies to glass slides and staining with Wright-Geimsa. As mentioned above, eosinophils are determined by staining with Luxol Fast Blue (Johnson, G. and Metcalf, D., Exp. Hematol. Vol. 8, pgs. 549-561 [1980]).

## D. BCGF II Activity.

For mice, a B cell growth factor known as B cell growth factor II (BCGF II) has been defined on the basis of two separate assays: (1) the ability to stimulate normal purified B cells to proliferate when exposed to dextran sulfate, and (2) the ability to augment in vitro proliferation of the in vivo-passaged $BCl_1$-lymphoma cells: Swain and Dutton, J. Exp. Med., Vol. 158, pg. 21 (1982); and Swain et al., J. Exp. Med., Vol. 158, pg. 822 (1982). These are important references for the descriptions of the BCGF II assays. $BCl_1$ cells are available from the ATCC under accession number TIB 197, and are described in Nature Vol. 272, pgs. 624-626 (1978), in Immuno. Rev., Vol. 48, pgs. 169-195 (1979), and in J. Immunol., Vol. 125, pgs. 976-980 (1980).

For the dextran sulfate-based BCGF II assay, normal B cells are prepared as described above and then passed through a Sephadex G-10 column. Cells are then cultured (as described above) at a concentration of about 5 $\times$ $10^4$ in 0.1 ml per well (of microtiter plates) with serial dilutions of the test sample. The degree of proliferation is determined at various times afterwards by pulse labeling with a radioactive precursor, e.g. by addition of $^{125}$I-uridine 6 hours prior to harvesting. Preferably cells are allowed to grow for at least 72 hours before adding the radioactive precursor.

For the $BCl_1$-based assay, spleens are removed from the mice (preferably BALB/cByJ or BALB/cJ) bearing the $BCl_1$ tumor (cell recoveries varied from 8 $\times$ $10^8$ to 1.3 $\times$ $10^9$ per mouse) and are treated in vitro with Thy-1.2 monoclonal antibodies and anti-Lyt-2.2 monoclonal antibodies plus complement, as for normal B cells. The resulting cell population is resuspended at 5 $\times$ $10^5$/ml, and 5 $\times$ $10^4$ cells are added to microtiter cultures in 0.1 ml of culture medium. Test samples are added in 10 or 20 $\mu$l vol. Proliferation is determined at various times as described above for normal B cells. It is believed that $BCl_1$ cells are also responsive to human BCGF IIs, and therefore can serve as the basis for a human BCGF II assay.

## V. Purification and Pharmaceutical Compositions.

The polypeptides of the present invention expressed in E. coli, in yeast or in other cells can be purified according to standard procedures of the art, including ammonium sulfate precipitation, fractionation column chromatography (e.g., ion exchange, gel filtration, electrophoresis, affinity chromatography, etc.) and ultimately crystallization (see generally "Enzyme Purification and Related Techniques," Methods in Enzymology, 22:233-577 [1977] and Scopes, R., Protein Purification: Principles and Practice, Springer-Verlag, New York [1982]). Once purified, partially or to homogeneity, the polypeptides of the invention may be used for research purposes, e.g., as a supplement to cell growth media (e.g., minimum essential medium Eagle, Iscove's modified Dulbecco Medium or RPMI 1640, available from Sigma Chemical Company (St. Louis, MO) and GIBCO Division (Chagrin Falls, OH)) and as an antigenic substance for eliciting specific immunoglobulins useful in immunoassays, immunofluorescent stainings, etc. (See generally Immunological Methods, Vols. I & II, Eds. Lefkovits, I. and Pernis, B., Academic Press, New York, N.Y. [1979 & 1981], and Handbook of Experimental Immunology, ed. Weir, D., Blackwell Scientific Publications, St. Louis, MO [1978].)

The polypeptides of the present invention may also be used in pharmaceutical compositions, e.g., to enhance natural defense against various infections. Thus, patients suffering from rheumatoid arthritis or needing a transplant, or suffering from immunodeficiency caused by cancer chemotherapy, advanced age, immunosuppressive agents, etc., may be treated with such polypeptides. The compositions can selectively stimulate various components of the immune system, either alone or with other agents well known to those skilled in the art.

For preparing pharmaceutical compositions containing the polypeptides described by this invention, these polypeptides are compounded by admixture with preferably inert, pharmaceutically acceptable carriers. Suitable carriers and processes for their preparation are well known in the art (see, e.g., Remington's Pharmaceutical Sciences and U.S. Pharmacopeia: National Formulary, Mack Publishing Company, Easton, PA [1984]). The preferred course of administration is parenteral and can include use of mechanical delivery systems.

Preferably, the pharmaceutical composition is in unit dosage form. In such form, the preparation is subdivided into unit doses containing appropriate quantities of the active component. The quantity of active compound in a unit dose of preparation may be varied or adjusted from 1 μg to 100 mg, according to the particular application and the potency of the active ingredient. The composition can, if desired, also contain other therapeutic agents.

The dosages may be varied depending upon the requirement of the patient, the severity of the condition being treated and the particular compound being employed. The term "effective amount" as used herein is meant to take these factors into account when dosages are considered. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached. For convenience, the total daily dosage may be divided and administered in portions during the day.

## VI. Expression Systems

Once the cDNA of the invention has been cloned, a wide range of expression systems (i.e. host-expression vector combinations) can be used to produce the proteins of the invention. Possible types of host cells include, but are not limited to, bacterial, yeast, insect, mammalian, and the like. Selecting an expression system, and optimizing protein production thereby, requires the consideration and balancing of many factors, including (1) the nature of the protein to be expressed, e.g. the protein may be poisonous to some host organisms, it may be susceptible to degradation by host proteases, or it may be expressed in inactive conformations or in insoluble form in some hosts, (2) the nature of the messenger RNA (mRNA) corresponding to the protein of interest, e.g. the mRNA may have sequences particularly susceptible to host endonucleases, which drastically reduce the functional lifetime of the mRNA, or the mRNA may form secondary structures that mask the start codon or ribosome binding site, thereby inhibiting initiation of translation in some hosts, (3) the selection, availability, and arrangement of host-compatible expression control sequences in the 3'- and 5'-regions flanking the coding region -- these include promoters, 5'- and 3'-protector sequences, ribosome binding sites, transcription terminators, enhancers, polyadenylate addition sites, cap sites, intron-splice sites, and the like, (4) whether the protein has a secretion signal sequence which can be processed by the host, or whether an expression control sequence encoding a signal sequence endogenous to the host must be spliced onto the region encoding the mature protein, (5) the available modes and efficiencies of transfection or transformation of the host, and whether transient or stable expression is desired, (6) the scale and cost of the host culture system desired for expressing the protein, (7) the type of posttranslational modifications that may be desired, e.g. the extent and kind of glycosylation desired may affect the choice of host: see Uy and Wold, Science, Vol. 198, pgs. 890-896 (1977), (8) the ease with which the expressed protein can be separated from proteins and other materials of the host cells and/or culture medium e.g. in some cases it may be desirable to express a fusion protein with a specialized signal sequence to aid in later purification steps, (9) the stability and copy number of a particular vector in a selected host, e.g. Hofschneider et al., eds. Gene Cloning in Organisms Other than E. Coli (Springer Verlag, Berlin, 1982), and (10) like factors known to those skilled in the art.

Many reviews are available which provide guidance for making choices and/or modifications of specific expression systems in the light of the recited factors, e.g. to name a few, de Boer and Shepard in "Strategies for Optimizing Foreign Gene Expression in Escherichia coli," pgs. 205-247, in Kroon, ed. Genes: Structure and Expression (John Wiley & Sons, New York, 1983), review several E. coli expression systems; McClure in "Mechanism and Control of Transcription Initiation in Prokaryotes," Ann. Rev. Biochem. Vol. 54, pgs. 171-204 (1985) reviews prokaryotic promoters generally; Platt in Ann. Rev. Biochem., Vol. 55, pgs. 339-372 (1986) reviews transcription termination in prokaryotes and eukaryotes; Kucherlapati et al. in Critical Reviews in Biochemistry, Vol. 16, Issue 4, pgs. 349-379 (1984), and Banerji et al. in Genetic Engineering, Vol. 5, pgs. 19-31 (1983) review methods for transfecting and transforming mammalian cells; Oliver in Ann. Rev. Microbiol., Vol. 39, pgs. 615-648 (1985) reviews protein secretion in E. coli; and Campbell, in Ann. Rev. Biochem., Vol. 55, pgs. 733-771 (1986) reviews DNA replication of several vectors compatible with eukaryotic hosts.

Likewise, many reviews are available which describe techniques and conditions for linking and/or manipulating specific cDNAs and expression-control sequences to create and/or modify expression vectors suitable for use with the present invention, e.g. Maniatis et al., Molecular Cloning: A Laboratory Manual (Cold Spring Harbor Laboratory, N.Y., 1982), Glover, DNA Cloning: A Practical Approach, Vol. I and II (IRL Press, Oxford, 1985), and Perbal, A Practical Guide to Molecular Cloning (John Wiley & Sons, N.Y., 1984), to name only a few. Generally, within an expression vector various sites may be selected for insertion of the cDNA of the invention. These sites are usually designated by the restriction endonuclease which cuts them and are well recognized by those of skill in the art. Various methods for inserting DNA sequences into these sites to form recombinant DNA molecules are also well known. These include, for example, dG-dC or dA-dT tailing, direct ligation, synthetic linkers, exonuclease- and polymerase-linked repair reactions followed by ligation, or extension of the DNA strand with DNA polymerase and an appropriate single-stranded template followed by ligation.

Often a vector containing the cDNA of the invention must be obtained in large quantities before transfection and/or transformation of cells in a host culture can take place. For this purpose the vector is often replicated without significant expression in an organism (the cloning host) other than the one finally used for expression. In such cases, after propagation, the vectors are separated from the cloning host using standard techniques, e.g. as disclosed by Maniatis et al. (cited above).

Suitable prokaryote expression vectors include plasmids from E. coli, e.g. Col E1, pCR1, pBR322, pMB9 and their derivatives, plasmids suitable for a wider range of hosts, e.g. RP4, phage DNAs such as phage lambda and its various derivatives, M13, and the like. Additional E. coli vectors are described in Chapter 12 of Maniatis et al. (cited above) for expressing eukaryotic proteins, either unfused or fused with prokaryotic peptides. Riggs discloses further E. coli expression systems in U.S. Patent 4,431,739.

Commonly used prokaryotic promoters include the β-lactamase (penicillinase) and lactose promoter systems (Chang et al., Nature Vol. 275, pg. 615 (1978); Itakura et al., Science, Vol. 198, pg. 1056 (1977); Goeddel et al., Nature Vol. 281, pg. 544 (1979); and the tryptophan (trp) promoter system (Goeddel et al., Nucleic Acids Res., Vol. 8, pg. 4057 (1980); EPO Appl Publ No. 0036776). Whereas these are the most commonly used, other microbial promoters have been discovered and utilized, and details concerning their nucleotide sequences have been published, enabling a skilled worker to ligate them functionally with plasmid vectors, e.g. Siebenlist et al., Cell, Vol. 20, pg. 269 (1980).

A particularly useful prokaryotic promoter for high expression in E. coli is the tac promoter, disclosed by deBoer in U.S. Patent 4,551,433. Secretion expression vectors are also available for E. coli hosts. Particularly useful are the pIN-III-ompA vectors, disclosed by Ghrayeb et al. in EMBO J., Vol. 3, pgs. 2437-2442 (1984), in which the cDNA to be transcribed is fused to the portion of the E. coli OmpA gene encoding the signal peptide of the ompA protein which, in turn, causes the mature protein to be secreted into the periplasmic space of the bacteria. Likewise, U.S. Patents 4,336,336 and 4,338,397 disclose particularly useful secretion expression vectors for prokaryotes.

Numerous stains of bacteria are suitable hosts for prokaryotic expression vectors including strains of E. coli, such as W3110 (ATCC No. 27325), JA221, C600, ED767, DH1, LE392, HB101, X1776 (ATCC No. 31244), X2282, RR1 (ATCC No. 31343) MRCl; strains of Bacillus subtilus; and other enterobacteriaceae such as Salmonella typhimurium or Serratia marcescens, and various species of Pseudomas. General methods for deriving bacterial strains, such as E. coli K12 X1776, useful in the expression of eukaryotic proteins, are disclosed by Curtis III in U.S. Patent 4,190,495.

Eukaryotic microbes such as yeast cultures can also be used to express proteins of the invention. Saccharomyces cerevisiae, or common baker's yeast, is the most commonly used among eukaryotic microorganisms, although a number of other strains are commonly available. For expression in Saccharomyces, the plasmid YRp7 can be used, e.g. Stinchcomb et al., Nature, Vol. 282, pg. 39 (1979), Kingsman et al., Gene, Vol. 7, pg. 141 (1979), and Tschemper et al., Gene, Vol. 10, pg. 157 (1980). This plasmid already contains the trp1 gene which provides a selection marker for a mutant strain of yeast lacking the ability to grow in tryptophan, for example ATCC No. 44076 or PEP4-1 (Jones, Genetics, Vol. 85, pg. 12 (1977)). The presence of the trp1 lesion as a characteristic of the yeast host cell genome then provides an effective environment for detecting transformation by growth in the absence of tryptophan. Additional vectors for yeast include the pGAL plasmids disclosed by Miyajima et al., Nucleic Acids Research, Vol. 12, pgs. 6397-6414 (1984), and the 2μm plasmid disclosed by Beggs, Nature, Vol. 275, pgs. 104-109 (1978).

Suitable promoting sequences in yeast vectors include the promoters for 3-phosphoglycerate kinase (Hitzeman et al., J. Biol. Chem., Vol. 255, pg. 2073 (1980)) or other glycolytic enzymes (Hess et al, J. Adv. Enzyme Reg., Vol. 7, pg. 149 (1968); Holland et al., Biochemistry, Vol. 17, pg. 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triose-phosphate isomerase, phosphoglucose isomerase, and glucokinase. In constructing suitable expression plasmids, the termination sequences associated with these genes are also ligated into the expression vector adjacent to the 3'-end of the sequence to be expressed to provide polyadenylation and termination. Other promoters, which have the additional advantage of transcription controlled by growth conditions are the promoter regions for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization (Holland, cited above). Yet another regulatable promoter is the important metallothionein promoter system disclosed by Fogel et al. in U.S. Patent 4,511,652. Virtually any plasmid vector containing a promoter, an origin of replication and termination sequences that are all yeast-compatible is suitable.

Secretion expression vectors are also available for S. cerevisiae hosts, e.g. pMFα8 disclosed by Miyajima et al., Gene Vol. 37, pgs. 155-161 (1985); YEpIPT disclosed by Hitzeman et al., Science, Vol. 219, pgs. 620-625 (1983); pYαEGF-21 disclosed by Brake et al., Proc. Natl. Acad. Sci., Vol. 81, pgs. 4642-4646 (1984) and by Brake in European Patent Application 0116201; and plasmids disclosed by Singh in European Patent Application 0123544.

In addition to prokaryotic and eukaryotic microorganisms, expression systems comprising cells derived from multicellular organisms may also be used to produce proteins of the invention. Of particular interest are mammalian expression systems because their posttranslational processing machinery is more likely to produce biologically active mammalian proteins. Several DNA tumor viruses have been used as vectors for mammalian hosts; e.g. see Tooze, ed., DNA Tumor Viruses, 2nd Ed. (Cold Spring Harbor Laboratory, N.Y., 1981) for a review of their biology. Particularly important are the numerous vectors which comprise SV40-replication, -transcription, and/or -translation control sequences coupled to bacterial replication control sequences, e.g. the pcD vectors developed by Okayama and Berg, disclosed in Mol. Cell. Biol., Vol. 2, pgs. 161-170 (1982) and Mol. Cell. Biol., Vol. 3, pgs. 280-289 (1983); the SV40 vectors disclosed by Hamer in Genetic Engineering, Vol. 2, pgs. 83-100 (1980) and in U.S. Patent 4,599,308; and the vectors additionally containing adenovirus regulatory elements, disclosed by Kaufman and Sharp, in Mol. Cell. Biol., Vol. 2, pgs. 1304-1319 (1982), and by Kaufman et al. in European Patent Application 8406107. Monkey cells are usually the preferred hosts for the above vectors. Such vectors containing the SV40 ori sequences and an intact A gene can replicate autonomously in monkey cells (to give higher copy numbers and/or more stable copy numbers than nonautonomously replicating plasmids). Moreover, vectors containing the SV40 ori sequences without an intact A gene can replicate autonomously to high copy numbers (but not stably) in COS7 monkey cells, described by Gluzman, Cell, Vol. 23, pgs. 175-182 (1981) and available from the ATCC (accession no. CRL 1651). The above SV40-based vectors are also capable of transforming other mammalian cells, such as mouse L cells, by integration into the host cell DNA.

Besides the SV40-based vectors, mammalian expression systems suitable for use with the present invention include, but are not limited to, (i) vectors comprising bovine papilloma virus (BPV) sequences, e.g. BPV-pBR322 hybrids disclosed by Sarver et al., Genetic Engineering, Vol. 5, pgs. 173-190 (1983); and by DiMaio et al., Proc. Natl. Acad. Sci., Vol. 79, pgs. 4030-4034 (1982) for transforming bovine and mouse cells; (ii) vectors comprising Epstein-Barr virus (EBV) sequences, e.g. plasmids carrying the EBV oriP sequences (including the coding sequences for the nuclear antigen EBNA-1) disclosed by Yates et al., Nature, Vol. 313, pgs. 812-815 (1985); Reisman et al., Mol. Cell. Biol., Vol. 5, pgs. 1822-1832 (1985); Yates et al., Proc. Natl. Acad. Sci., Vol. 81, pgs. 3806-3810 (1984); and Sugden et al., Mol. Cell. Biol., Vol. 5, pgs. 410-413 (1985) for stable transformation of various mammalian cells including human and monkey cells; (iii) vectors comprising murine polyoma virus sequences for transforming mouse and hamster cells, e.g. O'Hara, J. Mol. Biol., Vol. 151, pg. 203 (1981); (iv) vectors carrying the dihydrofolate reductase (dhfr) gene, e.g. Alt et al., J. Biol. Chem., Vol. 253, pgs. 1357-1370 (1978), which in response to methotrexate treatment duplicates together with adjacent coding regions cointegrated into murine genomes (e.g., that of a Chinese hamster ovary (CHO) cell line deficient in dhfr activity) e.g. as described by Urlamb et al., Proc. Natl. Acad. Sci., Vol. 77, pg. 4216 (1980); and (v) cotransformation systems such as that disclosed by Axel et al. in U.S. Patent 4,399,216. Additional mammalian expression vectors can be constructed, or existing ones modified, by using various elements from available DNA tumor viruses and retroviruses, e.g. origins of replication, enhancer sequences (such as the long terminal repeat sequence from Rous sarcoma virus (RSV-LTR) disclosed by Gorman et al., Proc. Natl. Acad. Sci., Vol. 79, pgs. 6777-6781 (1982)) intron-splice sites, polyadenylation sites, and the like.

Invertebrate expression systems can also be constructed for use with the invention, e.g. the larvae of silkworm, Bombyx mori, infected by a baculovirus vector, BmNPV, described by Maeda et al. in Nature Vol. 315, pgs. 892-894 (1985); and in Saibo Koguku, Vol. 4, pgs. 767-779 (1985).

EXAMPLES

The following examples serve to illustrate the present invention. Selection of vectors and hosts as well as the concentration of reagents, temperatures, and the values of other variables are only to exemplify application of the present invention and are not to be considered limitations thereof.

Example I. Construction of cDNA Library from Cl.Lyl+2-/9 Cells, Preparation of Pool 114, and Transient Expression of PIF in COS 7 Monkey Cells

cDNA clones of pool 114 were isolated from a cDNA library constructed from the murine helper T cell line Cl.Lyl+2−/9, which is deposited with the ATCC under accession number CRL 8179 and described by Nabel et al. in Cell, Vol. 23, pgs. 19-28 (1981), and in Proc. Natl. Acad. Sci., Vol. 78, pgs. 1157-1161 (1981). Briefly, a pcD cDNA library was constructed with messenger RNA (mRNA) from concanvalin A (conA) -induced Cl.Lyl+2−/9 cells following the procedure of Okayama and Berg, described above.

A. Induction of PIF Production.

Cl.Lyl+2−/9 cells were induced to produce PIF mRNA by ConA as follows. The cells were cultured at 5 × $10^5$/ml in Dulbecco's Modified Eagles medium (DME) with 4% heat-inactivated fetal calf serum, 5 × $10^{-5}$ M 2-mercaptoethanol (2-ME), 2mM glutamine, non-essential amino acids, essential vitamins and 2 µg/ml ConA. After 12-14 hrs. incubation at 37°C in 10% $CO_2$, the cell suspension was centrifuged at 1500 rpm for 10 minutes. The cell pellets were collected and frozen immediately at -70°C.

B. Isolation of mRNA

Total cellular DNA was isolated from cells using the guanidine isothiocyanate procedure of Chirgwin J. et al. (Biochemistry, 18:5294-5299 [1979]). Frozen cell pellets from ConA-induced Cl.Lyl+2−/9 cells (12 hrs after stimulation) were suspended in guanidine isothiocyanate lysis solution. Twenty ml of lysis solution was used for 1.5 × $10^8$ cells. Pellets were resuspended by pipetting, and then DNA was sheared by 4 passes through a syringe using a 16 gauge needle. The lysate was layered on top of 20 ml of 5.7 M CsCl, 10 mM EDTA in a 40 ml polyallomer centrifuge tube. This solution was centrifuged at 25,000 rpm in a Beckman SW28 rotor (Beckman Instruments, Inc., Palo Alto, CA) for 40 hrs at 15°C. The guanidine isothiocyanate phase containing DNA was pipetted off from the top, down to the interface. The walls of the tube and interface were washed with 2-3 ml of guanidine isothiocyanate lysis solution. The tube was cut below the interface with scissors, and the CsCl solution was decanted. RNA pellets were washed twice with cold 70% ethanol. Pellets were then resuspended in 500 µl of 10 mM Tris.HCl pH 7.4, 1 mM EDTA, 0.05% SDS. 50 µl of 3M sodium acetate was added and RNA was precipitated with 1 ml ethanol. About 0.3 mg total RNA was collected by centrifuging and the pellets washed once with cold ethanol.

Washed and dried total RNA pellet was resuspended in 900 µl of oligo (dT) elution buffer (10 mM Tris.HCl, pH 7.4, 1 mM EDTA, 0.5% SDS). RNA was heated for 3 min. at 68°C and then chilled on ice. 100 µl of 5 M NaCl was added. The RNA sample was loaded onto a 1.0 ml oligo (dT) cellulose column (Type 3, Collaborative Research, Watham, MA) equilibrated with binding buffer (10 mM Tris.HCl pH 7.4, 1 mM EDTA, 0.5 M NaCl, 0.5% SDS). Flow-through from the column was passed over the column twice more. The column was then washed with 20 ml binding buffer. PolyA+ mRNA was collected by washing with elution buffer. RNA usually eluted in the first 2 ml of elution buffer. RNA was precipitated with 0.1 volume 3 M sodium acetate (pH 6) and two volumes of ethanol. The RNA pellet was collected by centrifugation, washed twice with cold ethanol, and dried. The pellet was then resuspended in water. Aliquots were diluted, and absorbance at 260 nm was determined.

C. Construction of pcD cDNA Library

1) Preparation of Vector Primer and Oligo(dG)-Tailed Linker DNAs.

The procedure of Okayama & Berg (Mol. & Cell. Biol. Vol. 2, pgs.161-170 [1982]) was used with only minor modifications. The pcDV1 and pL1 plasmids are described by Okayama & Berg (Mol. & Cell. Biol. 3:380-389 [1983]) and are available from Pharmacia (Piscataway, N.J.). Specifically, a modified pcDV1 plasmid was used which contained an Nsil site at the previous location of the Kpnl site.

An 80 µg sample of pcDV1 DNA was digested at 30°C with 20 U of Kpnl endonuclease in a reaction mixture of 450 µl containing 6 mM Tris.HCl (pH 7.5), 6 mM MgCl₂, 6 mM NaCl, 6 mM 2-ME, and 0.1 mg of bovine serum albumin (BSA) per ml. After 16 hr the digestion was terminated with 40 µl of 0.25 M EDTA (pH 8.0) and 20 µl of 10% sodium dodecyl sulfate (SDS); the DNA was recovered after extraction with water-saturated 1:1 phenol-CHCl₃ (hereafter referred to as phenol-CHCl₃) and precipitation with ethanol. Homopolymer tails averaging 60, but not more than 80, deoxythymidylate (dT) residues per end were added to the Nsil endonuclease-generated termini with calf thymus terminal transferase as follows: The reaction mixture (38 µl) contained sodium cacodylate-30 mM Tris.HCl pH 6.8 as buffer, with 1 mM CoCl₂, 0.1 mM dithiothreitol, 0.25 mM dTTP, the Nsil endonuclease-digested DNA, and 68 U of the terminal deoxynucleotidyl transferase (P-L Biochemicals, Inc., Milwaukee, WI). After 30 min. at 37°C the reaction was stopped with 20 µl of 0.25 M EDTA (pH 8.0) and 10 µl of 10% SDS, and, after several extractions with phenol-CHCl₃, the DNA was recovered by precipitation with ethanol. The DNA was then digested with 15 U of EcoRI endonuclease in 50 µl containing 10 mM Tris.HCl pH 7.4, 10 mM MgCl₂, 1 mM dithiothreitol, and 0.1 mg of BSA per ml for 5 hr at 37°C. The large fragment, containing the SV40 polyadenylation site, the pBR322 origin of replication and the ampicillin-resistance gene, was purified by agarose (1%) gel electrophoresis and recovered from the gel by a modification of the glass powder method (Vogelstein, B. & Gillespie, D., Proc. Natl. Acad. Sci. 76: 615-619 [1979]). The dT-tailed DNA was further purified by absorption and elution from an oligo (dA)-cellulose column as follows: The DNA was dissolved in 1 ml of 10 mM Tris.HCl pH 7.3 buffer containing 1 mM EDTA and 1 M NaCl, cooled to 0°C, and applied to an oligo (dA)-cellulose column (0.6 by 2.5 cm) equilibrated with the same buffer at 0°C and eluted with water at room temperature. The eluted DNA was precipitated with ethanol and dissolved in 10 mM Tris.HCl pH 7.3 with 1 mM EDTA.

The oligo (dG)-tailed linked DNA was prepared by digesting 75 μg of pL1 DNA with 20 U of Pstl endonuclease in 450 μl containing 6 mM Tris.HCl pH 7.4, 6 mM MgCl₂, 6 mM 2-ME, 50 mM NaCl, and 0.01 mg of BSA per ml. After 16 hr at 30°C the reaction mixture was extracted with phenol-CHCl₃ and the DNA was precipitated with alcohol. Tails of 10 to 15 deoxyguanylate (dG) residues were then added per end with 46 U of terminal deoxynucleotidyl transferase in the same reaction mixture (38 μl) as described above, except that 0.1 mM dGTP replaced dTTP. After 20 min. at 37°C the mixture was extracted with phenol-CHCl₃, and the DNA was precipitated with ethanol and then digested with 35 U of HindIII endonuclease in 50 μl containing 20 mM Tris.HCl pH 7.4, 7 mM MgCl₂, 60 mM NaCl, and 0.1 mg of BSA at 37°C for 4 hr. The small oligo (dG)-tailed linker DNA was purified by agarose gel (1.8%) electrophoresis and recovered as described above.

2) cDNA Library Preparation:

Step 1: cDNA synthesis. The reaction mixture (10 μl) contained 50 mM Tris.HCl pH 8.3, 8 mM MgCl₂, 30 mM KCl, 0.3 mM dithiothreitol, 2 mM each dATP, dTTP, dGTP, and dCTP, 20 μCi $^{32}$P-dCTP (3000 Ci/mmole), 3 μg polyA+ RNA from ConA-induced T cells, 60 units RNasin (a tradenamed ribonuclease inhibitor from Promega Biotec, Inc., Madison, WI), and 2 μg of the vector-primer DNA (15 pmol of primer end), and 45 U of reverse transcriptase. The reaction was incubated 60 min at 42°C and then stopped by the addition of 1 μl of 0.25 M ETDA (pH 8.0) and 0.5 μl of 10% SDS; 40 μl of phenol-CHCl₃ was added, and the solution was blended vigorously in a Vortex mixer and then centrifuged. After addition of 40 μl of 4 M ammonium acetate and 160 μl of ethanol to the aqueous phase, the solution was chilled with dry ice for 15 min., warmed to room temperature with gentle shaking to dissolve unreacted deoxynucleoside triphosphates that had precipitated during chilling, and centrifuged for 10 min. in an Eppendorf microfuge. The pellet was dissolved in 10 μl of 10 mM Tris.HCl pH 7.3 and 1 mM EDTA, mixed with 10 μl of 4 M ammonium acetate, and reprecipitated with 40 μl of ethanol, a procedure which removes more than 99% of unreacted deoxynucleotide triphosphates. The pellet was rinsed with ethanol.

Step 2: Oligodeoxycytidylate [oligo (dC)] addition. The pellet containing the plasmid-cDNA:mRNA was dissolved in 20 μl of 140 mM sodium cacodylate-30 mM Tris.HCl pH 6.8 buffer containing 1 mM CoCl₂, 0.1 mM dithiothreitol, 0.2 μg of poly (A), 70 μM dCTP, 5 uCi $^{32}$P-dCTP, 3000 Ci/mmole, and 60 U of terminal deoxynucleotidyl transferase. The reaction was carried out at 37°C for 5 min. to permit the addition of 10 to 15 residues of dCMP per end and then terminated with 2 μl of 0.25 M EDTA (pH 8.0) and 1 μl of 10% SDS. After extraction with 20 μl of phenol-CHCl₃, the aqueous phase was mixed with 20 μl of 4 M ammonium acetate, the DNA was precipitated and reprecipitated with 80 μl of ethanol, and the final pellet was rinsed with ethanol.

Step 3: HindIII endonuclease digestion. The pellet was dissolved in 30 μl of buffer containing 20 mM Tris.HCl pH 7.4, 7 mM MgCl₂, 60 mM NaCl, and 0.1 mg of BSA per ml and then digested with 10 U of HindIII endonuclease for 2 hr at 37°C. The reaction was terminated with 3 μl of 0.25 M EDTA (pH 8.0) and 1.5 μl of 10% SDS and, after extraction with phenol-CHCl₃ followed by the addition of 30 μl of 4 M ammonium acetate, the DNA was precipitated with 120 μl of ethanol. The pellet was rinsed with ethanol and then dissolved in 10 μl of 10 mM Tris.HCl (pH 7.3) and 1 mM EDTA, and 3 μl of ethanol was added to prevent freezing during storage at -20°C.

Step 4: Cyclization mediated by the oligo (dG)-tailed linker DNA. A 9 μl sample of the HindIII endonuclease-digested oligo (dC)-tailed cDNA:mRNA plasmid (about 90% of the sample) was incubated in a mixture (90 μl) containing 10 mM Tris.HCl pH 7.5, 1 mM EDTA, 0.1 M NaCl, and 1.8 pmol of the oligo (dG)-tailed linker DNA at 65°C for 5 min., shifted to 42°C for 60 min, and then cooled to 0°C. The mixture (90 μl) was adjusted to a volume of 900 μl containing 20 mM Tris.HCl pH 7.5, 4 mM MgCl₂, 10 mM (NH₄)₂SO₄, 0.1 M KCl, 50 μg of BSA per ml, and 0.1 mM β-NAD; 6 μg of E. coli DNA ligase were added and the solution was then incubated overnight at 12°C.

Step 5: Replacement of RNA strand by DNA. To replace the RNA strand of the insert, the ligation mixture was adjusted to contain 40 μM of each of the four deoxynucleoside triphosphates, 0.15 mM beta-NAD, 4 μg of additional E. coli DNA ligase, 16 U of E. coli DNA polymerase I (PolI,) and 9U of E. coli RNase H. This mixture (960 μl) was incubated successively at 12°C and at room temperature for 1 hr each to promote optimal repair synthesis and nick translation by PolI.

Step 6: Transformation of E. coli. Transformation was carried out using minor modifications of the procedure described by Cohen et al. (Proc. Nat. Acad. Sci. U.S.A., Vol. 69, pgs. 2110-2114 [1972]). E. coli K-12 strain MC1061 (Casadaban, M. and Cohen, S., J. Mol. Biol. Vol. 138, pgs. 179-207 [1980]) was grown to 0.5 absorbancy unit at 600 nm at 37°C in 300 ml of L-broth. The cells were collected by centrifugation, suspended in 30 ml of 10 mM Pipes (pH 7), 60 mM CaCl₂, 15% glycerol and centrifuged at 0°C for 5 min. The cells were resuspended in 24 ml of the above buffer and incubated again at 0°C for 5 min.; then, 1.0 ml aliquots of the cell suspensions were mixed with 0.1 ml of the DNA solution (step 5) and incubated at 0°C for 20 min. Next the cells were kept at 42°C for 2 min. and thereafter at room temperature for 10 min.; then 1 liter of L-broth was added, and the culture was incubated at 37°C for 60 min.

Ampicillin was added to a concentration of 50 micrograms/ml, and the culture was shaken for an additional 10 hrs. at 37°C. Samples were then taken from the culture and frozen down in DMSO for storage. Separately an aliquot of the culture was diluted to about 4300 cells/ml (determined by test plating a 0.1 ml sample and counting colonies). About 30 pools were established from 0.125 ml samples from this aliquot, and then amplified to form colonies containing 537 clones with a standard deviation of 23. Plasmid DNA was isolated from the amplified cultures of each pool using standard techniques (e.g. Maniatis et al., cited above), and used to transfect COS 7 cells as follows.

One day prior to transfection, approximately $10^6$ COS 7 monkey cells were seeded onto individual 100 mm plates in DME containing 10% fetal calf serum and 2 mM glutamine. To perform the transfection, the medium was aspirated from each plate and replaced with 4 ml of DME containing 50 mM Tris.HCl pH 7.4, 400 µg/ml DEAE-Dextran and 50 µg of the plasmid DNAs to be tested. The plates were incubated for four hours at 37°C, then the DNA-containing medium was removed, and the plates were washed twice with 5 ml of serum-free DME. DME containing 150 µM Chloroquine was added back to the plates which were then incubated for an additional 3 hrs at 37°C. The plates were washed once with DME, and then DME containing 4% fetal calf serum, 2 mM glutamine, penicillin and streptomycin was added. The cells were then incubated for 72 hrs at 37°C. The growth medium was collected and evaluated for Eo-CSF and IgA-EF activities. Plasmids from pool 114 produced positive responses in both assays.

Example II. Screening Pool 114 by Functional Cloning to Isolate a cDNA Encoding a Polypeptide Exhibiting PIF Activity

An individual cDNA clone capable of encoding a polypeptide exhibiting PIF activity was isolated from pool 114 by subdividing it into 20 subpools, each containing about 160 distinct clones. The subpools were amplified, and plasmid DNA was prepared from each. The isolated plasmid DNA was then used to transfect COS 7 cells as described above, and COS 7 culture supernatants were assayed for IgA-EF and Eo-CSF activities to identify positive subpools. A positive subpool was further subdivided until a single clone, designated clone 4G, was obtained. Supernatants of COS 7 cells transfected with clone 4G exhibited Eo-CSF activity and IgA-EF activity.

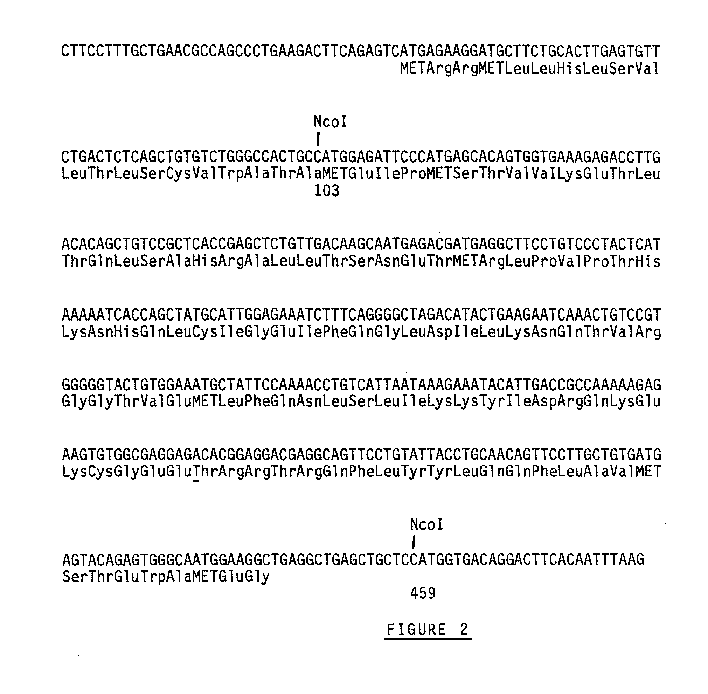

The cDNA insert of clone 115 was re-cloned into an M13 plasmid and sequenced using the dideoxy chain termination method; e.g. Sanger et al., Proc. Natl. Acad. Sci., Vol. 74, pgs. 5363-5367 (1977). The sequence is illustrated in Figure 2.

Example III. Purification of Murine PIF from Culture Supernatants of COS 7 Monkey Cells Transfected by cDNA Clones of Pool 114.

COS 7 cells are transfected with plasmid from pool 114 as described in Example I, and supernatants are collected and concentrated by passage through YM-10 Amicon membrane (to give a protein concentration of about 12 mg/ml), and dialyzed against 20 mM NaCl in a dialysis buffer (DB) consisting of 50 mM Hepes pH 7.0, 10% glycerol, 1 mM EDTA, 1 mM EGTA, 0.5 microgram/ml leupeptin, 0.5 microgram/ml Pepstatin A, 0.5 mM phenylmethylsulfonyl fluoride (PMSF), and 0.04% Tween 20. The dialysate in DB plus 20 mM NaCl is then loaded onto a heparin-agarose column (Pharmacia) at about 300 mg protein per 250 ml column volume. Before loading, the column is equilibrated against DB plus 20 mM NaCl. The PIF is followed by BCDF activity as defined in the assay (not requiring a B cell mitogen) described above. After loading, and washing with DB plus 20 mM NaCl, the PIF is eluted from the column stepwise in DB plus 150 mM NaCl. The eluted protein is concentrated with a YM-10 Amicon membrane to a protein concentration of 10 mg/ml and chromatographed through two serially coupled prepacked Superose-12 columns (Pharmacia) pre-equilibrated in DB plus 400 mM NaCl. Active material eluted at an apparent molecular weight of 8 kilodaltons (kDs). It is believed, however, that this apparent molecular weight is an artifact due to the hydrophobic interaction of PIF with the column matrix. Subsequent analysis by SDS-PAGE indicates a molecular weight in the range of about 40-48 kD. The active fractions are again concentrated by a YM-10 Amicon membrane, and then applied to a C-4 reverse phase column (Altec). A piecewise linear gradient of acetonitrile in 0.1% (v/v) trifluoroacetic acid (TFA) is run through the column at a flow rate of 0.75 ml/minute. After injection, the column is washed with 0.1% TFA in $H_2O$ for 10 minutes, after which acetonitrile concentration is increased linearly from 0% to 40% (v/v) over a 2 minute period, and then held constant for 5 minutes. After five minutes at 40% acetonitrile, the concentration is increased from 40% to 80% acetonitrile over a 40 minute period, after which acetonitrile concentration is increased to 100% in a single step. PIF elutes at about 60% acetonitrile and is immediately placed in a mixture of 50 mM Hepes pH 8.0, 20% glycerol, 0.1% Tween 20 to preserve activity. The eluted sample of PIF is purified about $2 \times 10^4$-fold with respect to BCDF activity.

Example IV. Preparation of Human PIF Via a Murine cDNA Probe to a Human Helper T Cell cDNA Library and Transient Expression in COS7 Monkey Cells.

A cDNA clone coding for PIF was isolated from a cDNA library constructed from induced peripheral blood lymphocytes (PBLs). Other cell sources for cDNA libraries include helper T cell clones, e.g. such as 2FI described by Yokota et al. Proc. Natl. Acad. Sci., Vol. 83, pgs. 5894-5898 (1986), or variants of the CEM line, available from the ATCC under accession numbers CCL 119, CRL 8436, and TIB 195, and described by Foley et al. in Cancer, Vol. 18, pgs. 522-529 (1965), and by Ligler in Lymphokine Research, Vol. 3, pgs. 183-191 (1984).

PBLs were grown in Iscove's medium supplemented with 3% fetal calf serum. The PBLs were induced by adding 1 microgram/ml ConA to their cultures. Cells are harvested about 10 hours after the addition of ConA.

mRNA extraction and cDNA library construction are carried out as described in Example I. The NcoI fragment (shown in Figure 2) containing the murine PIF cDNA was isolated from murine pcD-mPIF, labeled by nick translation ($1 \times 10^8$ cpm/µg) and used to probe nitrocellulose filters containing plasmid DNA preparations from ten pools, each representing approximately $1 \times 10^3$ clones from the PBL cDNA library. Low stringency hybridization conditions (overnight at 42°C) were used: $6 \times$ SSPE ($1 \times$ SSPE = 180 mM NaCl/10mM sodium phosphate, pH 7.4/1 mM EDTA) (Maniatis, T. et al., Molecular Cloning: A Laboratory Manual (Cold Spring Harbor Laboratory, N.Y., 1982)), 20% (vol/vol) formamide, 0.1% sodium dodecyl sulfate, yeast carrier tRNA at 100µl. The filters are washed with 2 × SSPE, 0.1% sodium dodecyl sulfate at 37°C.

A human PIF cDNA was detected on a filter, and the corresponding clone, designated "clone 115", was isolated from the culture plate. Clone 115 was amplified in E. coli MC1061 and transfected into COS 7 monkey cells. Supernatants of the transfected monkey cells exhibited Eo-CSF activity (using human progenitor cells) and BCDF activity (using SAC-activated purified mouse B cells and measuring IgM production using standard ELISA techniques).

The cDNA insert of clone 115 was sequenced using the dideoxy chain termination method, and the putative native PIF amino acid sequence was determined by comparison with the mouse sequence, and application of the empirical rules, published by Perlman et al., J. Mol. Biol., Vol. 167, pgs. 391-409 (1983), for determining secretion signal sequences of polypeptides. The native human PIF amino acid sequence is given by Formula II.

Example V. Construction of a Synthetic Human PIF Gene for Cassette Mutagenesis in pcD and Expression in COS 7 Monkey Cells.

Techniques for constructing and expressing the synthetic gene of this example are standard in the art of molecular biology, e.g. especially Sproat and Gait, Nucleic Acids Research, Vol. 13, pgs. 2959-2977 (1985), and Ferretti et al., Proc. Natl. Acad. Sci., Vol. 83, pgs. 599-603 (1986); and also Mullenbach et al., J. Biol. Chem., Vol. 261, pgs. 719-722 (1986), Wells et al., Gene, Vol. 34, pgs. 315-323 (1985), and Estell et al., Science, Vol. 233, pgs. 659-663 (1986). Briefly, the synthetic human PIF gene is assembled from a plurality of chemically synthesized double-stranded DNA fragments. Base sequences of the synthetic gene are selected so that the assembled synthetic gene contains a series of unique restriction sites with respect to the vector carrying the gene.

The series of unique restriction sites defines a series of segments which can be readily excised and replaced with segments having altered base sequences. The synthetic fragments are inserted either directly or after ligation with other fragments into a suitable vector, such as a pcD plasmid, or the like. The above-mentioned segments correspond to the "cassettes" of Wells et al. (cited above). The synthetic fragments are synthesized using standard techniques, e.g. Gait, Oligonucleotide Synthesis: A Practical Approach (IRL Press, Oxford, UK, 1984). Preferably an automated synthesizer is employed, such as an Applied Biosystems, Inc. (Foster City, CA) model 380A. pcD plasmids and other suitable vectors are commercially available, e.g. from Pharmacia. For pcD, cloning can be carried out in E. coli MC1061 or HB101, and expression can be carried out in COS monkey cells, Chinese hamster ovary cells, or mouse L cells.

In this example a synthetic human PIF gene is constructed for insertion into a pcD plasmid. Briefly, the pcD vector is constructed by first ligating four fragments together with T4 DNA ligase: the large HindIII/BamHI fragment from pcDV1 containing the pBR322 ori and Ampr, the HindIII/PstI fragment from pL1 containing the SV40 early region promoter and late region introns, and synthetics 1A/B and 2A/B, described below. Next, in a series of three additional amplification, purification, and insertion steps the rest of the synthetics 3A/B through 7A/B are added until a complete synthetic PIF gene is present in the pcD vector.

Restriction endonuclease digestions and ligase reactions are performed using standard protocols, e.g. Maniatis et al., Molecular Cloning: A Laboratory Manual (Cold Spring Harbor Laboratory, New York, 1982).

The alkaline method (Maniatis et al., cited above) is used for small-scale plasmid preparations. For large scale preparations a modification of the alkaline method is used in which an equal volume of isopropanol is used to precipitate nucleic acids from the cleared lysate. Precipitation with cold 2.5 M ammonium acetate is used to remove RNA prior to separation by equilibrium density centrifugation in cesium chloride and detection with ethidium bromide.

Complementary strands of synthetic DNAs to be cloned (about 400ng each) are mixed and phosphorylated with polynucleotide kinase in a reaction volume of 50µl. This DNA is ligated with 1µg of vector DNA digested with appropriate restriction enzymes, and ligations are in a volume of 50 µl at room temperature for 4 to 12 hours. Conditions for phosphorylation, restriction enzyme digestions, polymerase reactions, ligations, and bacterial transfections have been described (Maniatis et al., cited above).

In step 1, pcDV1 is amplified, isolated, and digested with HindIII and BamHI. The fragment containing the pBR322 ori and Ampr regions is isolated by gel electrophoresis using standard techniques, e.g. of Maniatis et al. (cited above). pL1 is amplified, isolated and digested with HindIII and PstI. The fragment containing the SV40 early region promoter is isolated by gel electrophoresis. The two isolated pcDV1 and pL1 fragments are then mixed with synthetics 1A/B and 2A/B (illustrated below) in a standard T4 DNA ligase solution. Synthetics 1A/B and 2A/B anneal to form an insert to the newly created pcD vector, which is amplified in E. coli and then isolated.

In step 2, the isolated pcD vector of step 1 is digested with NheI and SacI. The large fragment is separated and mixed with synthetics 3A/B and 4A/B in a standard T4 DNA ligase solution to form the pcD vector of step 2, which is amplified in E. coli MC1061 and isolated.

In step 3, the isolated pcD vector of step 2 is digested with XbaI and SacI. The large fragment is separated and mixed with synthetics 5A/B and 6A/B in a standard T4 DNA ligase solution to form the pcD vector of step 3, which is amplified in E. coli MC1061 and isolated.

In step 4, the isolated pcD vector of step 3 is digested with MluI and SacI. The large fragment is separated and mixed with synthetics 7A/B in a standard T4 DNA ligase solution to form the pcD vector containing the complete synthetic human PIF gene. This pcD vector is amplified in E. coli MC1061, isolated, and transfected into COS 7 monkey cells to express PIF.

The sequences of synthetics 1A/B through 7A/B are listed below. Locations and types of unique restriction sites are indicated above the sequences. Lower case letters indicate where base alterations are made in the native nucleotide sequence.

```
(PstI)  ApaI
        GGGGCCCATGAGGATGCTTCTG-
ACGTCCCCGGGTACTCCTACGAAGAC-


                        NarI
CATTTGAGTTTaCTAGCTCTTGGcGC-
GTAAACTCAAAtGATCGAGAACCgCG-


cGCCTACGTGTAT
gCGG
```

Synthetics 1A/B

```
        GCCATCCCCACAGAAATT-
ATGCACATACGGTAGGGGTGTCTTTAA-


    SpeI
CCCACtAGTGCATTGGTGAAAGAGACg-
GGGTGaTCACGTAACCACTTTCTCTGc-


NheI  SacI  (HindIII)
cTaGCGAGCTCA
gAtCGCTCGAGTTCGA
```

Synthetics 2A/B

NheI
cTaGCACTGCTTTCTACTCATCGAACT-
    GTGACGAAAGATGAGTAGCTTGA-

                        SnaBI
CTGGTGATAGCCAATGAGACTCTacGt-
GACCACTATCGGTTACTCTGAGAtgCa-

ATTCCTGTTCCT
TAA


<u>Synthetics 3A/B</u>


                 GTACATAAAAATCACCAA-
GGACAAGGACATGTATTTTTAGTGGTT-

            BglII
CTGTGCACTGAAGAgATCTTTCAGGGA-
GACACGTGACTTCTcTAGAAAGTCCCT-


       XbaI    (SacI)
ATAGGCACTCTAGAGAGCt
TATCCGTGAGATCTC


<u>Synthetics 4A/B</u>

XbaI
CTaGAGAGTCAAACTGTGCAAGGG-
    TCTCAGTTTGACACGTTCCC-


GGTACTGTGGAAAGACTATTCAAA-
CCATGACACCTTTCTGATAAGTTT-

           BclI

```
AACTTGTCCTTg
TTG
```

## Synthetics 5A/B

        BclI

```
          ATcAAGAAATACATTGAC-
AACAGGAAcTAgTTCTTTATGTAACTG-


GGCCAAAAAAAAAAGTGTGGAGAAGAA-
CCGGTTTTTTTTTTTCACACCTCTTCTT-
```

        MluI   (SacI)

```
AGACGacGcGTGAGCT
TCTGCtgCgCAC
```

## Synthetics 6A/B

  MluI

```
cGcGTAAACCAATTCCTAGACTACCTG-
    ATTTGGTTAAGGATCTGATGGAC-
```

        EcoRI

```
CAAGAaTTcCTTGTTGTAATGAACACC-
GTTCTtAAgGAACAACATTACTTGTGG-
```

               (SacI)

```
GAGTGGATAATAGAAAGTTAGGAGCT
CTCACCTATTATCTTTCAATCC
```

## Synthetics 7A/B

Example VI. Construction and Expression of Human PIF Mutein Ile[7].

Leu at position 7 is changed to Ile to form human PIF mutein Ile[7]. The pcD plasmid of Example V containing the complete synthetic PIF gene is digested with SpeI and NheI. The large fragment is isolated and combined with the following synthetic in a standard T4 DNA ligase solution:

```
CTAGTGCAaTaGTGAAAGAGACG
ACGTtAtCACTTTCTCTGCGATC
```

The resulting pcD vector is amplified in E. coli MC1061, isolated, and transfected into COS 7 monkey cells in accordance with the protocols described above. After incubation for 3-4 days, COS 7 culture supernatants are harvested and assayed for PIF activity.

Example VII. Construction and Expression of Human PIF Mutein Val[23].

Ile at position 23 is changed to Val to form human PIF mutein Val[23]. The pcD vector of Example V containing the complete synthetic PIF gene is digested with NheI and SanBI. The large fragment is isolated and combined with the following synthetic in a standard T4 DNA ligase solution:

```
CTAGCACTGCTTTCTACTCATCG-
GTGACGAAAGATGAGTAGA-
```

```
AACTCTGCTGgTAGCCAATGAGACT-
TTGAGACGACcATCGGTTACTCTGA-
```

```
CTAC
GATG
```

The resulting pcD vector is amplified in E. coli MC1061, isolated, and transfected into COS 7 monkey cells in accordance with the protocols described above. After incubation for 3-4 days, COS 7 culture supernatants are harvested and assayed for PIF activity.

Example VIII. Construction and Expression of Human PIF Mutein Glu[47].

Gln at position 47 is changed to Glu to form human PIF mutein Glu[47]. The pcD vector of Example V containing the complete synthetic PIF gene is digested with BglII and XbaI. The large fragment is isolated and combined with the following synthetic in a standard T4 DNA ligase solution:

```
GATCTTTgAGGGAATAGGCACT
AAAcTCCCTTATCCGTGAGATC
```

The resulting pcD vector is amplified in E. coli MC1061, isolated, and transfected into COS 7 monkey cells in accordance with the protocols described above. After incubation for 3-4 days, COS 7 culture supernatants are harvested and assayed for PIF activity.

Example IX. Construction and Expression of Human PIF Mutein Leu[104].

Met at position 104 is changed to Leu to form human PIF mutein Leu[104]. The pcD vector of Example V containing the complete synthetic PIF gene is digested with EcoRI and SacI. The large fragment is isolated and combined with the following synthetic in a standard T4 DNA ligase solution:

```
AATTCCTTGGTGTATtGAACACC-
GGAACCACATaACTTGTGG-
```

```
GAGTGGATAATAGAAAGTTAGGACCT
CTCACCTATTATCTTTCAATCC
```

The resulting pcD vector is amplified in E. coli MC1061, isolated, and transfected into COS 7 monkey cells in accordance with the protocols described above. After incubation for 3-4 days, COS 7 culture supernatants are harvested and assayed for PIF activity.

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to best utilize the invention in various embodiments and with various modifications as are suited to any particular use.

cDNA pool 114 has been deposited with the American Type Culture Collection, Rockville, MD, USA (ATCC), under accession number 67267.

**Claims**

1. A protein exhibiting eosinophil colony stimulating factor activity or B cell differentiation factor activity comprising a glycosylated or unglycosylated 1-10-fold substituted polypeptide selected from the set defined by the formula:

```
X(Glu) - X(Ile) - X(Pro) - X(Thr) - X(Ser) - X(Ala) -
                            10
X(Leu) - X(Val) - X(Lys) - X(Glu) - X(Thr) - X(Leu) -

X(Ala) - X(Leu) - X(Leu) - X(Ser) - X(Thr) - X(His) -
            20
X(Arg) - X(Thr) - X(Leu) - X(Leu) - X(Ile) - X(Ala) -
                                        30
X(Asn) - X(Glu) - X(Thr) - X(Leu) - X(Arg) - X(Ile) -

X(Pro) - X(Val) - X(Pro) - X(Val) - X(His) - X(Lys) -
                            40
X(Asn) - X(His) - X(Gln) - X(Leu) - X(Cys) - X(Thr) -

X(Glu) - X(Glu) - X(Ile) - X(Phe) - X(Gln) - X(Gly) -
            50
X(Ile) - X(Gly) - X(Thr) - X(Leu) - X(Glu) - X(Ser) -
                                        60
X(Gln) - X(Thr) - X(Val) - X(Gln) - X(Gly) - X(Gly) -

X(Thr) - X(Val) - X(Glu) - X(Arg) - X(Leu) - X(Phe) -
                            70
X(Lys) - X(Asn) - X(Leu) - X(Ser) - X(Leu) - X(Ile) -

X(Lys) - X(Lys) - X(Tyr) - X(Ile) - X(Asp) - X(Gly) -
            80
X(Gln) - X(Lys) - X(Lys) - X(Lys) - X(Cys) - X(Gly) -
                                        90
X(Glu) - X(Glu) - X(Arg) - X(Arg) - X(Arg) - X(Val) -

X(Asn) - X(Gln) - X(Phe) - X(Leu) - X(Asp) - X(Tyr) -


                            100
X(Leu) - X(Gln) - X(Glu) - X(Phe) - X(Leu) - X(Gly) -

X(Val) - X(Met) - X(Asn) - X(Thr) - X(Glu) - X(Trp) -
            110
X(Ile) - X(Ile) - X(Glu) - X(Ser)
```

wherein:

X(Ser) is Ser;

X(Arg) represents Arg, His, or Lys;

X(Leu) represents Leu, Ile, Phe, or Met;

X(Pro) represents Pro or Ala;

X(Thr) is Thr;

X(Ala) represents Ala or Pro;

X(Val) represents Val, Met, or Ile;

X(Gly) is Gly;

X(Ile) represents Ile, Met, Phe, Val, or Leu;

X(Phe) represents Phe, Met, Tyr, Ile, or Leu;

X(Tyr) represents Tyr or Phe;

X(His) represents His, Gln, or Arg;

X(Gln) represents Gln, Glu, or His;

X(Asn) represents Asn or Asp;

X(Lys) represents Lys or Arg;

X(Asp) represents Asp or Asn;

X(Glu) represents Glu or Gln;

X(Met) represents Met, Phe, Ile, Val, or Leu;

X(Cys) is Cys; and

X(Trp) is Trp.

2. The protein of claim 1 wherein said polypeptide is 5-fold substituted, preferably 2-fold substituted.

3. The protein of claim 1 wherein:

X(Arg) is Arg;

X(Leu) represents Leu, Ile, or Met;

X(Pro) is Pro;

X(Ala) is Ala;

X(Val) is Val;

X(Ile) represents Ile, Met, or Leu;

X(Phe) is Phe;

X(Tyr) is Tyr;

X(His) is His;

X(Gln) is Gln;

X(Asn) is Asn;

X(Lys) is Lys;

X(Asp) is Asp;

X(Glu) is Glu; and

X(Met) represents Met, Ile, or Leu;

especially said protein which is 5-fold substituted, preferably 2-fold substituted.

4. The protein of claim 3 wherein said polypeptide comprises a sequence of amino acids defined by the formula:

```
Glu - Ile - Pro - Thr - Ser - Ala - Leu - Val - Lys -
10
Glu - Thr - Leu - Ala - Leu - Leu - Ser - Thr - His -
       20
Arg - Thr - Leu - Leu - Ile - Ala - Asn - Glu - Thr -
          30
Leu - Arg - Ile - Pro - Val - Pro - Val - His - Lys -
                40
Asn - His - Gln - Leu - Cys - Thr - Glu - Glu - Ile -
```

50
Phe – Gln – Gly – Ile – Gly – Thr – Leu – Glu – Ser –

60
Gln – Thr – Val – Gln – Gly – Gly – Thr – Val – Glu –

70
Arg – Leu – Phe – Lys – Asn – Leu – Ser – Leu – Ile –

80
Lys – Lys – Tyr – Ile – Asp – Gly – Gln – Lys – Lys –

90
Lys – Cys – Gly – Glu – Glu – Arg – Arg – Arg – Val –

Asn – Gln – Phe – Leu – Asp – Tyr – Leu – Gln – Glu –
100
Phe – Leu – Gly – Val – Met – Asn – Thr – Glu – Trp –
110
Ile – Ile – Glu – Ser.

5. A protein exhibiting eosinophil colony stimulating factor activity or B cell differentiation factor activity comprising a glycosylated or
unglycosylated 0-5-fold inserted, 0-5-fold deleted, and 1-5-fold substituted polypeptide having a sequence of amino acids selected from the set defined by the formula:

X(Glu) – X(Ile) – X(Pro) – X(Thr) – X(Ser) – X(Ala) –

10
X(Leu) – X(Val) – X(Lys) – X(Glu) – X(Thr) – X(Leu) –

X(Ala) – X(Leu) – X(Leu) – X(Ser) – X(Thr) – X(His) –
20
X(Arg) – X(Thr) – X(Leu) – X(Leu) – X(Ile) – X(Ala) –

30
X(Asn) – X(Glu) – X(Thr) – X(Leu) – X(Arg) – X(Ile) –

X(Pro) – X(Val) – X(Pro) – X(Val) – X(His) – X(Lys) –

27

0 267 779

```
                                        40
X(Asn) - X(His) - X(Gln) - X(Leu) - X(Cys) - X(Thr) -

X(Glu) - X(Glu) - X(Ile) - X(Phe) - X(Gln) - X(Gly) -
                 50
X(Ile) - X(Gly) - X(Thr) - X(Leu) - X(Glu) - X(Ser) -
                                                  60
X(Gln) - X(Thr) - X(Val) - X(Gln) - X(Gly) - X(Gly) -

X(Thr) - X(Val) - X(Glu) - X(Arg) - X(Leu) - X(Phe) -
                                  70
X(Lys) - X(Asn) - X(Leu) - X(Ser) - X(Leu) - X(Ile) -

X(Lys) - X(Lys) - X(Tyr) - X(Ile) - X(Asp) - X(Gly) -
                 80
X(Gln) - X(Lys) - X(Lys) - X(Lys) - X(Cys) - X(Gly) -
                                                  90
X(Glu) - X(Glu) - X(Arg) - X(Arg) - X(Arg) - X(Val) -

X(Asn) - X(Gln) - X(Phe) - X(Leu) - X(Asp) - X(Tyr) -
                          100
X(Leu) - X(Gln) - X(Glu) - X(Phe) - X(Leu) - X(Gly) -

X(Val) - X(Met) - X(Asn) - X(Thr) - X(Glu) - X(Trp) -
                 110
X(Ile) - X(Ile) - X(Glu) - X(Ser)
```

wherein:

    X(Ser) is Ser;
    X(Arg) represents Arg, His, or Lys;
    X(Leu) represents Leu, Ile, Phe, or Met;
    X(Pro) represents Pro or Ala;
    X(Thr) is Thr;
    X(Ala) represents Ala or Pro;
    X(Val) represents Val, Met, or Ile;
    X(Gly) is Gly;
    X(Ile) represents Ile, Met, Phe, Val, or Leu;
    X(Phe) represents Phe, Met, Tyr, Ile, or Leu;
    X(Tyr) represents Tyr or Phe;
    X(His) represents His, Gln, or Arg;
    X(Gln) represents Gln, Glu, or His;
    X(Asn) represents Asn or Asp;
    X(Lys) represents Lys or Arg;
    X(Asp) represents Asp or Asn;
    X(Glu) represents Glu or Gln;
    X(Met) represents Met, Phe, Ile, Val, or Leu;
    X(Cys) is Cys; and
    X(Trp) is Trp.

6. The protein of claim 5 wherein said polypeptide is 2-fold inserted, 2-fold deleted, and 2-fold substituted, preferably 1-fold inserted, 1-fold deleted, and 1-fold substituted.

7. A nucleic acid cassette of from 10 to 120 bases in length comprising a nucleotide sequence selected from the set of nucleotide sequences capable of encoding a segment of a 10-fold substituted polypeptide selected from the set defined by the formula:

X(Glu) – X(Ile) – X(Pro) – X(Thr) – X(Ser) – X(Ala) –

10

X(Leu) – X(Val) – X(Lys) – X(Glu) – X(Thr) – X(Leu) –

X(Ala) – X(Leu) – X(Leu) – X(Ser) – X(Thr) – X(His) –

20

X(Arg) – X(Thr) – X(Leu) – X(Leu) – X(Ile) – X(Ala) –

30

X(Asn) – X(Glu) – X(Thr) – X(Leu) – X(Arg) – X(Ile) –

X(Pro) – X(Val) – X(Pro) – X(Val) – X(His) – X(Lys) –

40

X(Asn) – X(His) – X(Gln) – X(Leu) – X(Cys) – X(Thr) –

X(Glu) – X(Glu) – X(Ile) – X(Phe) – X(Gln) – X(Gly) –

50

X(Ile) – X(Gly) – X(Thr) – X(Leu) – X(Glu) – X(Ser) –

60

X(Gln) – X(Thr) – X(Val) – X(Gln) – X(Gly) – X(Gly) –

X(Thr) – X(Val) – X(Glu) – X(Arg) – X(Leu) – X(Phe) –

70

X(Lys) – X(Asn) – X(Leu) – X(Ser) – X(Leu) – X(Ile) –

X(Lys) – X(Lys) – X(Tyr) – X(Ile) – X(Asp) – X(Gly) –

80

X(Gln) – X(Lys) – X(Lys) – X(Lys) – X(Cys) – X(Gly) –

90

X(Glu) – X(Glu) – X(Arg) – X(Arg) – X(Arg) – X(Val) –

X(Asn) – X(Gln) – X(Phe) – X(Leu) – X(Asp) – X(Tyr) –

100

X(Leu) – X(Gln) – X(Glu) – X(Phe) – X(Leu) – X(Gly) –

X(Val) – X(Met) – X(Asn) – X(Thr) – X(Glu) – X(Trp) –

110

X(Ile) – X(Ile) – X(Glu) – X(Ser)

wherein:

    X(Ser) is Ser;
    X(Arg) represents Arg, His, or Lys;
    X(Leu) represents Leu, Ile, Phe, or Met;
    X(Pro) represents Pro or Ala;
    X(Thr) is Thr;
    X(Ala) represents Ala or Pro;
    X(Val) represents Val, Met, or Ile;

29

X(Gly) is Gly;
X(Ile) represents Ile, Met, Phe, Val, or Leu;
X(Phe) represents Phe, Met, Tyr, Ile, or Leu;
X(Tyr) represents Tyr or Phe;
X(His) represents His, Gln, or Arg;
X(Gln) represents Gln, Glu, or His;
X(Asn) represents Asn or Asp;
X(Lys) represents Lys or Arg;
X(Asp) represents Asp or Asn;
X(Glu) represents Glu or Gln;
X(Met) represents Met, Phe, Ile, Val, or Leu;
X(Cys) is Cys; and
X(Trp) is Trp.

8. The nucleic acid cassette of claim 7 wherein said nucleotide sequence is selected from a set of nucleotide sequences capable of encoding a segment of a 2-fold substituted polypeptide selected from the set defined by the formula:

```
X(Glu) - X(Ile) - X(Pro) - X(Thr) - X(Ser) - X(Ala) -
                              10
X(Leu) - X(Val) - X(Lys) - X(Glu) - X(Thr) - X(Leu) -
X(Ala) - X(Leu) - X(Leu) - X(Ser) - X(Thr) - X(His) -
             20
X(Arg) - X(Thr) - X(Leu) - X(Leu) - X(Ile) - X(Ala) -
                                                 30
X(Asn) - X(Glu) - X(Thr) - X(Leu) - X(Arg) - X(Ile) -
X(Pro) - X(Val) - X(Pro) - X(Val) - X(His) - X(Lys) -
                              40
X(Asn) - X(His) - X(Gln) - X(Leu) - X(Cys) - X(Thr) -
X(Glu) - X(Glu) - X(Ile) - X(Phe) - X(Gln) - X(Gly) -
             50
X(Ile) - X(Gly) - X(Thr) - X(Leu) - X(Glu) - X(Ser) -
                                                 60
X(Gln) - X(Thr) - X(Val) - X(Gln) - X(Gly) - X(Gly) -
X(Thr) - X(Val) - X(Glu) - X(Arg) - X(Leu) - X(Phe) -
                              70
X(Lys) - X(Asn) - X(Leu) - X(Ser) - X(Leu) - X(Ile) -
X(Lys) - X(Lys) - X(Tyr) - X(Ile) - X(Asp) - X(Gly) -
```

$$X(Gln) - X(Lys) - X(Lys) - X(Lys) - X(Cys) - X(Gly) -$$

$$X(Glu) - X(Glu) - X(Arg) - X(Arg) - X(Arg) - X(Val) -$$

$$X(Asn) - X(Gln) - X(Phe) - X(Leu) - X(Asp) - X(Tyr) -$$

$$X(Leu) - X(Gln) - X(Glu) - X(Phe) - X(Leu) - X(Gly) -$$

$$X(Val) - X(Met) - X(Asn) - X(Thr) - X(Glu) - X(Trp) -$$

$$X(Ile) - X(Ile) - X(Glu) - X(Ser)$$

wherein:

X(Ser) is Ser;
X(Arg) represents Arg, His, or Lys;
X(Leu) represents Leu, Ile, Phe, or Met;
X(Pro) represents Pro or Ala;
X(Thr) is Thr;
X(Ala) represents Ala or Pro;
X(Val) represents Val, Met, or Ile;
X(Gly) is Gly;
X(Ile) represents Ile, Met, Phe, Val, or Leu;
X(Phe) represents Phe, Met, Tyr, Ile, or Leu;
X(Tyr) represents Tyr or Phe;
X(His) represents His, Gln, or Arg;
X(Gln) represents Gln, Glu, or His;
X(Asn) represents Asn or Asp;
X(Lys) represents Lys or Arg;
X(Asp) represents Asp or Asn;
X(Glu) represents Glu or Gln;
X(Met) represents Met, Phe, Ile, Val, or Leu;
X(Cys) is Cys; and
X(Trp) is Trp; in particular

where said nucleotide sequence is selected from the set of nucleotide sequences capable of encoding a segment of a polypeptide defined by the formula:

31

```
Glu - Ile - Pro - Thr - Ser - Ala - Leu - Val - Lys -
10
Glu - Thr - Leu - Ala - Leu - Leu - Ser - Thr - His -
        20
Arg - Thr - Leu - Leu - Ile - Ala - Asn - Glu - Thr -
        30
Leu - Arg - Ile - Pro - Val - Pro - Val - His - Lys -
                40
Asn - His - Gln - Leu - Cys - Thr - Glu - Glu - Ile -
                50
Phe - Gln - Gly - Ile - Gly - Thr - Leu - Glu - Ser -
                        60
Gln - Thr - Val - Gln - Gly - Gly - Thr - Val - Glu -
                        70
Arg - Leu - Phe - Lys - Asn - Leu - Ser - Leu - Ile -
                                80
Lys - Lys - Tyr - Ile - Asp - Gly - Gln - Lys - Lys -
                                90
Lys - Cys - Gly - Glu - Glu - Arg - Arg - Arg - Val -
Asn - Gln - Phe - Leu - Asp - Tyr - Leu - Gln - Glu -
100
Phe - Leu - Gly - Val - Met - Asn - Thr - Glu - Trp -
        110
Ile - Ile - Glu - Ser.
```

9. A nucleic acid comprising a nucleotide sequence selected from the set of nucleotide sequences capable of encoding the polypeptide defined by the formula:

```
Glu - Ile - Pro - Thr - Ser - Ala - Leu - Val - Lys -
10
Glu - Thr - Leu - Ala - Leu - Leu - Ser - Thr - His -
      20
Arg - Thr - Leu - Leu - Ile - Ala - Asn - Glu - Thr -
            30
Leu - Arg - Ile - Pro - Val - Pro - Val - His - Lys -
                  40
Asn - His - Gln - Leu - Cys - Thr - Glu - Glu - Ile -
                  50
Phe - Gln - Gly - Ile - Gly - Thr - Leu - Glu - Ser -
                        60
Gln - Thr - Val - Gln - Gly - Gly - Thr - Val - Glu -
                              70
Arg - Leu - Phe - Lys - Asn - Leu - Ser - Leu - Ile -
                                    80
Lys - Lys - Tyr - Ile - Asp - Gly - Gln - Lys - Lys -
                                          90
Lys - Cys - Gly - Glu - Glu - Arg - Arg - Arg - Val -
Asn - Gln - Phe - Leu - Asp - Tyr - Leu - Gln - Glu -
100
Phe - Leu - Gly - Val - Met - Asn - Thr - Glu - Trp -
      110
Ile - Ile - Glu - Ser.
```

10. A method of producing a polypeptide exhibiting B cell differentiation factor activity or eosinophil colony stimulating factor activity, the method comprising the steps of:

(a) constructing a vector comprising a nucleotide sequence coding for said polypeptide, wherein the nucleotide sequence is capable of being expressed by a host containing the vector and wherein the nucleotide sequence is selected from the group of nucleotide sequences capable of encoding a 10-fold substituted polypeptide defined by formula:

X(Glu) - X(Ile) - X(Pro) - X(Thr) - X(Ser) - X(Ala) -

10

X(Leu) - X(Val) - X(Lys) - X(Glu) - X(Thr) - X(Leu) -

X(Ala) - X(Leu) - X(Leu) - X(Ser) - X(Thr) - X(His) -

20

X(Arg) - X(Thr) - X(Leu) - X(Leu) - X(Ile) - X(Ala) -

30

X(Asn) - X(Glu) - X(Thr) - X(Leu) - X(Arg) - X(Ile) -

X(Pro) - X(Val) - X(Pro) - X(Val) - X(His) - X(Lys) -

40

X(Asn) - X(His) - X(Gln) - X(Leu) - X(Cys) - X(Thr) -

X(Glu) - X(Glu) - X(Ile) - X(Phe) - X(Gln) - X(Gly) -

50

X(Ile) - X(Gly) - X(Thr) - X(Leu) - X(Glu) - X(Ser) -

60

X(Gln) - X(Thr) - X(Val) - X(Gln) - X(Gly) - X(Gly) -

X(Thr) - X(Val) - X(Glu) - X(Arg) - X(Leu) - X(Phe) -

70

X(Lys) - X(Asn) - X(Leu) - X(Ser) - X(Leu) - X(Ile) -

X(Lys) - X(Lys) - X(Tyr) - X(Ile) - X(Asp) - X(Gly) -

80

X(Gln) - X(Lys) - X(Lys) - X(Lys) - X(Cys) - X(Gly) -

90

X(Glu) - X(Glu) - X(Arg) - X(Arg) - X(Arg) - X(Val) -

X(Asn) - X(Gln) - X(Phe) - X(Leu) - X(Asp) - X(Tyr) -

100

X(Leu) - X(Gln) - X(Glu) - X(Phe) - X(Leu) - X(Gly) -

X(Val) - X(Met) - X(Asn) - X(Thr) - X(Glu) - X(Trp) -

110

X(Ile) - X(Ile) - X(Glu) - X(Ser)

wherein:

X(Ser) is Ser;
X(Arg) represents Arg, His, or Lys;
X(Leu) represents Leu, Ile, Phe, or Met;
X(Pro) represents Pro or Ala;
X(Thr) is Thr;
X(Ala) represents Ala or Pro;
X(Val) represents Val, Met, or Ile;
X(Gly) is Gly;
X(Ile) represents Ile, Met, Phe, Val, or Leu;
X(Phe) represents Phe, Met, Tyr, Ile, or Leu;
X(Tyr) represents Tyr or Phe;
X(His) represents His, Gln, or Arg;
X(Gln) represents Gln, Glu, or His;
X(Asn) represents Asn or Asp;
X(Lys) represents Lys or Arg;
X(Asp) represents Asp or Asn;
X(Glu) represents Glu or Gln;
X(Met) represents Met, Phe, Ile, Val, or Leu;
X(Cys) is Cys; and
X(Trp) is Trp;

(b) incorporating the vector into the host; and

(c) maintaining the host in a culture medium under conditions suitable for expression of the nucleotide sequence into said polypeptide;

especially wherein said nucleotide sequence is selected from the nucleotide sequences capable of encoding a polypeptide defined by the formula:

```
Glu - Ile - Pro - Thr - Ser - Ala - Leu - Val - Lys -
10
Glu - Thr - Leu - Ala - Leu - Leu - Ser - Thr - His -
```

35

                        20
Arg – Thr – Leu – Leu – Ile – Ala – Asn – Glu – Thr –
                        30
Leu – Arg – Ile – Pro – Val – Pro – Val – His – Lys –
                        40
Asn – His – Gln – Leu – Cys – Thr – Glu – Glu – Ile –
                        50
Phe – Gln – Gly – Ile – Gly – Thr – Leu – Glu – Ser –
                        60
Gln – Thr – Val – Gln – Gly – Gly – Thr – Val – Glu –
                        70
Arg – Leu – Phe – Lys – Asn – Leu – Ser – Leu – Ile –
                        80
Lys – Lys – Tyr – Ile – Asp – Gly – Gln – Lys – Lys –
                        90
Lys – Cys – Gly – Glu – Glu – Arg – Arg – Arg – Val –
Asn – Gln – Phe – Leu – Asp – Tyr – Leu – Gln – Glu –
100
Phe – Leu – Gly – Val – Met – Asn – Thr – Glu – Trp –
110
Ile – Ile – Glu – Ser.

11. A pharmaceutical composition for stimulating the production of immunoglobulin comprising a therapeutically compatible carrier and an effective amount of a protein comprising a glycosylated or unglycosylated polypeptide having an amino acid sequence defined by the formula of any of claims 1-10, especially wherein the polypeptide has the formula:

```
Glu - Ile - Pro - Thr - Ser - Ala - Leu - Val - Lys -
10
Glu - Thr - Leu - Ala - Leu - Leu - Ser - Thr - His -
        20
Arg - Thr - Leu - Leu - Ile - Ala - Asn - Glu - Thr -
            30
Leu - Arg - Ile - Pro - Val - Pro - Val - His - Lys -
                40
Asn - His - Gln - Leu - Cys - Thr - Glu - Glu - Ile -
                    50
Phe - Gln - Gly - Ile - Gly - Thr - Leu - Glu - Ser -
                        60
Gln - Thr - Val - Gln - Gly - Gly - Thr - Val - Glu -
                            70
Arg - Leu - Phe - Lys - Asn - Leu - Ser - Leu - Ile -
                                80
Lys - Lys - Tyr - Ile - Asp - Gly - Gln - Lys - Lys -
                                    90
Lys - Cys - Gly - Glu - Glu - Arg - Arg - Arg - Val -
Asn - Gln - Phe - Leu - Asp - Tyr - Leu - Gln - Glu -
100
Phe - Leu - Gly - Val - Met - Asn - Thr - Glu - Trp -
        110
Ile - Ile - Glu - Ser.
```

12. A method for increasing levels of immunoglobulin isotype IgA in a mammal which comprises administering to said mammal an effective amount of a glycosylated or unglycosylated 0-10 fold substituted polypeptide of the formula defined in claim 1, or a protein defined in any of claims 2 to 6.

**FIGURE 1**

0267779

0267779

```
CTTCCTTTGCTGAACGCCAGCCCTGAAGACTTCAGAGTCATGAGAAGGATGCTTCTGCACTTGAGTGTT
                                      METArgArgMETLeuLeuHisLeuSerVal


                                 NcoI
                                  |
CTGACTCTCAGCTGTGTCTGGGCCACTGCCATGGAGATTCCCATGAGCACAGTGGTGAAAGAGACCTTG
LeuThrLeuSerCysValTrpAlaThrAlaMETGluIleProMETSerThrValValLysGluThrLeu
                                103


ACACAGCTGTCCGCTCACCGAGCTCTGTTGACAAGCAATGAGACGATGAGGCTTCCTGTCCCTACTCAT
ThrGlnLeuSerAlaHisArgAlaLeuLeuThrSerAsnGluThrMETArgLeuProValProThrHis


AAAAATCACCAGCTATGCATTGGAGAAATCTTTCAGGGGCTAGACATACTGAAGAATCAAACTGTCCGT
LysAsnHisGlnLeuCysIleGlyGluIlePheGlnGlyLeuAspIleLeuLysAsnGlnThrValArg


GGGGGTACTGTGGAAATGCTATTCCAAAACCTGTCATTAATAAAGAAATACATTGACCGCCAAAAAGAG
GlyGlyThrValGluMETLeuPheGlnAsnLeuSerLeuIleLysLysTyrIleAspArgGlnLysGlu


AAGTGTGGCGAGGAGACACGGAGGACGAGGCAGTTCCTGTATTACCTGCAACAGTTCCTTGCTGTGATG
LysCysGlyGluGluThrArgArgThrArgGlnPheLeuTyrTyrLeuGlnGlnPheLeuAlaValMET


                                 NcoI
                                  |
AGTACAGAGTGGGCAATGGAAGGCTGAGGCTGAGCTGCTCCATGGTGACAGGACTTCACAATTTAAG
SerThrGluTrpAlaMETGluGly
                                459
```

FIGURE 2